# EUROPEAN PATENT APPLICATION

(11) **EP 4 818 156 A1**
(43) Date of publication of application: **07.10.2026**
(21) Application number: 24906436.1
(22) Date of filing: 19.12.2024
(51) Int. Cl.: C07D 209/90, C07D 311/82, C08G 65/333, C09K 11/06, A61K 49/22

(54) **SQUARIC ACID COMPOUND, PHARMACEUTICALLY ACCEPTABLE SALT OR DEUTERATED COMPOUND THEREOF, PREPARATION METHOD THEREFOR AND USE THEREOF**

(30) Priority: 20.12.2023 CN 202311768625
(71) Applicant: Shanghai Institute of Materia Medica, Chinese Academy of Sciences, Shanghai 201203 (CN)
(72) Inventor: CHEN, Hao, Shanghai 201203 (CN); LEI, Peng, Shanghai 201203 (CN); SONG, Ruihu, Shanghai 201203 (CN); LEI, Meiling, Shanghai 201203 (CN)
(74) Representative: J A Kemp LLP
(86) International application number: PCT/CN2024/140501
(87) International publication number: WO 2025/130957

(57) **Abstract**

The present application relates to a squaric acid compound or a pharmaceutically acceptable salt or deuterated compound thereof, as well as a preparation method and use thereof. The squaric acid compound is represented by a general structural formula I, II, III, IV, V, or VI:

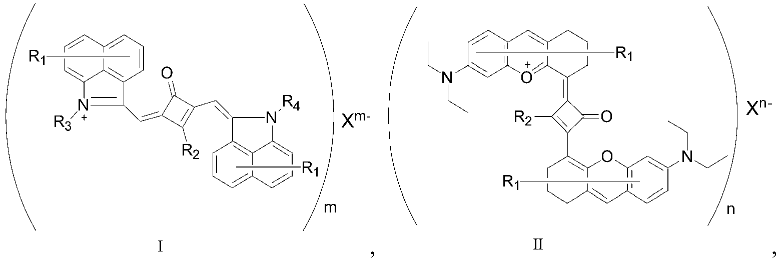

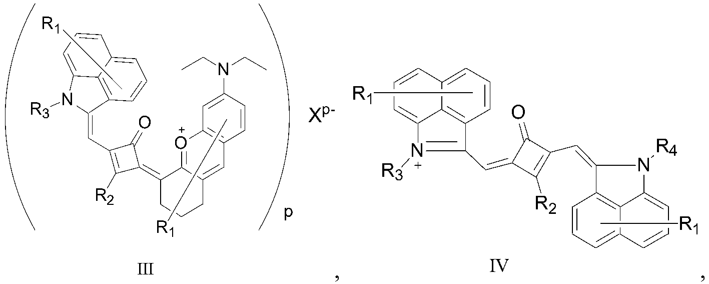

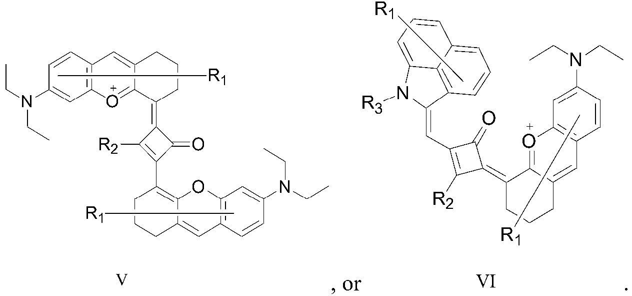

## Description

### RELATED APPLICATIONS

This application claims priority to the Chinese patent application No. 2023117686258, filed on December 20, 2023, entitled "SQUARIC ACID COMPOUND, PHARMACEUTICALLY ACCEPTABLE SALT OR DEUTERATED COMPOUND THEREOF, PREPARATION METHOD THEREFOR AND USE THEREOF", the content of which is incorporated herein by reference in its entirety.

### TECHNICAL FIELD

The present application relates to the field of organic fluorescent probes, and specifically relates to a squaric acid compound or a pharmaceutically acceptable salt or deuterated compound thereof, as well as a preparation method and use thereof.

### BACKGROUND

Optical imaging, especially fluorescence imaging that has developed rapidly and been widely applied in recent years, utilizes specific fluorescent molecular probes to label target molecules or cells, which has a spatial resolution reaching the millimeter scale. Widely known to life science researchers, optical imaging has been extensively used for *in vitro* imaging and well regarded by scientific personnel due to its advantages of relatively high sensitivity, rapidity and simplicity, low cost, and relatively high throughput. Critical challenges for optical imaging include autofluorescence, quenching, photobleaching, and low tissue penetration depth.

Compared with fluorescence imaging in the visible light region (400 to 700 nm), fluorescence imaging within the near-infrared (NIR) window (700 to 1700 nm) has considerable advantages in reducing photon scattering, lowering absorption, and minimizing autofluorescence interference *(*Chem. Soc. Rev., 2018, 47, 4258*).* Near-infrared imaging holds enormous potential owing to a high resolution and a high signal-to-noise ratio in molecular diagnosis and therapeutic applications. Over the past decade, near-infrared I fluorescence imaging (NIR-I, 700 to 900 nm) has been widely applied in basic research, as well as preclinical and clinical diagnosis, exemplified by FDA-approved indocyanine green (ICG) and methylene blue (MB). However, compared with NIR-I fluorescence imaging, NIR-II fluorescence imaging can mitigate tissue autofluorescence, reduce photon scattering, and lower photon absorption levels, enabling deeper penetration, and better imaging effect, signal-to-noise ratio, and sensitivity. Currently, NIR-II fluorescent imaging reagents for biological applications mainly include carbon nanotubes, quantum dots, rare-earth doped nanoparticles, small organic molecules, and conjugated polymers.

Compared with inorganic nanomaterials, small-molecule organic dyes have gained increasing attention from researchers, owing to their advantages including well-defined structures, low molecular weights, rapid metabolism, and safety. Researchers have attempted to extend the emission wavelengths of small-molecule dyes into the near-infrared region by optimizing structural design and synthetic routes. These diverse NIR dye structures enrich the NIR fluorescent probe library through the rational design of the main chain and substituents of the dye structures. For example, cyanine dyes and D-A-D type dyes exhibit favorable water solubility, quantum yield, and molar absorption coefficients in biological systems and tissues, and have been widely explored as NIR-II fluorescent probes. Nevertheless, cyanine dyes suffer from poor photostability, inferior chemical stability, low photothermal conversion efficiency, small Stokes shifts, and the fact that most developed dyes are confined to NIR-I. D-A-D type dyes show drawbacks such as high molecular weights, small molar absorption coefficients, or slow *in vivo* metabolic rates.

### SUMMARY

In view of this, the present application aims to provide a squaric acid compound, or a pharmaceutically acceptable salt or deuterated compound thereof, that emits near-infrared light, is photostable, has a low molecular weight, a large Stokes shift, and a large molar absorption coefficient, and can be used as a fluorescent probe and a near-infrared II contrast agent.

In an aspect, a squaric acid compound or a pharmaceutically acceptable salt or deuterated compound thereof is provided, wherein the squaric acid compound is represented by a general structural formula I, II, III, IV, V, or VI: 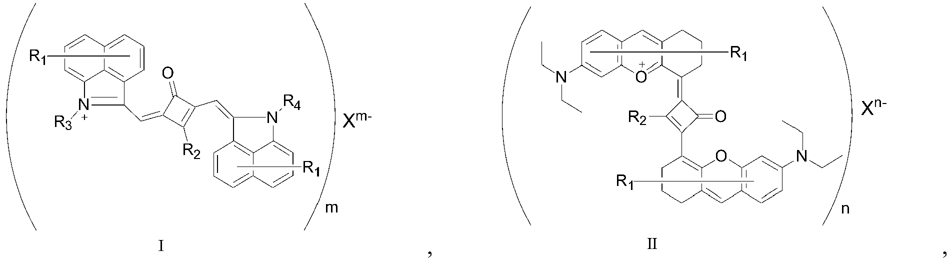 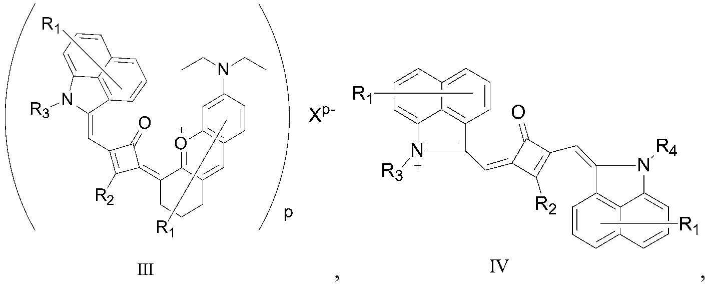 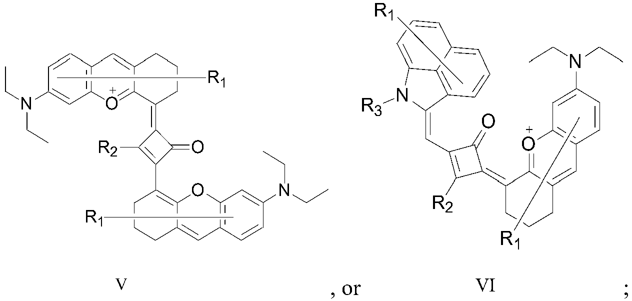
in which, X^{m-}, xⁿ⁻, and X^{p-} are each independently an anion,
m, n, and p are each independently any integer selected from 1 to 10;
each R₁ is independently at least one selected from the group consisting of H, C₁-C₁₂ alkyl, C₁-C₁₂ alkoxy, C₁-C₈ alkylsilyl, hydroxy C₁-C₈ alkyl, amino C₁-C₈ alkyl, formyl C₁-C₈ alkyl, mercapto C₁-C₈ alkyl, halo C₁-C₈ alkyl, acyloxy C₁-C₈ alkyl, amino, halogen, nitro, carboxy C₁-C₆ alkyl, substituted or unsubstituted C₆-C₁₀ aryl or 5 to 10 membered heteroaryl, -(CH₂)n₁-COOCH₂CH₂Si(CH₃)₃, -(CH₂)n₂-(CH₂CH₂O)n₃-R, and -(CH₂)n₂-(OCH₂CH₂)n₃-R;
R₂ is at least one selected from the group consisting of hydroxy, C₁-C₁₂ alkoxy, C₁-C₈ alkylsilyl, hydroxy C₁-C₈ alkyl, carboxy, amino, -NR₅R₆, mercapto, -SR₇, and malononitrile;
R₃, R₄, R₅, R₆, and R₇ are each independently at least one selected from the group consisting of H, C₁-C₈ alkyl, hydroxy, amino, carboxy, C₁-C₁₂ alkyl, C₁-C₁₂ alkoxy, C₁-C₈ alkylsilyl, hydroxy C₁-C₈ alkyl, amino C₁-C₈ alkyl, aldehyde C₁-C₈ alkyl, mercapto C₁-C₈ alkyl, halo C₁-C₈ alkyl, acyloxy C₁-C₈ alkyl, amino, halogen, sulfo, carboxy C₁-C₆ alkyl, substituted or unsubstituted C₆-C₁₀ aryl or 5 to 10 membered heteroaryl, -(CH₂)n₁-COOCH₂CH₂Si(CH₃)₃, -(CH₂)n₂-(CH₂CH₂O)n₃-R, and -(CH₂)n₂-(OCH₂CH₂)n₃-R;
n1 is any integer from 0 to 10, n2 is any integer from 0 to 10, and n3 is any integer from 1 to 500;
R is at least one selected from the group consisting of H, C₁-C₈ alkyl, hydroxy, amino, carboxy, sulfo, halogen, mercapto, 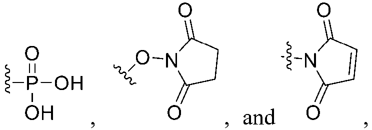 where 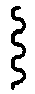 represents a linking site.

In an embodiment, the squaric acid compound is represented by a general structural formula I-1, II-1, III-1, IV-1, V-1, or VI-1:

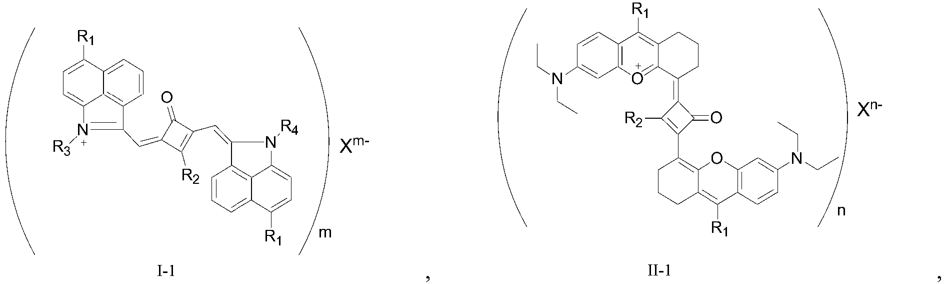

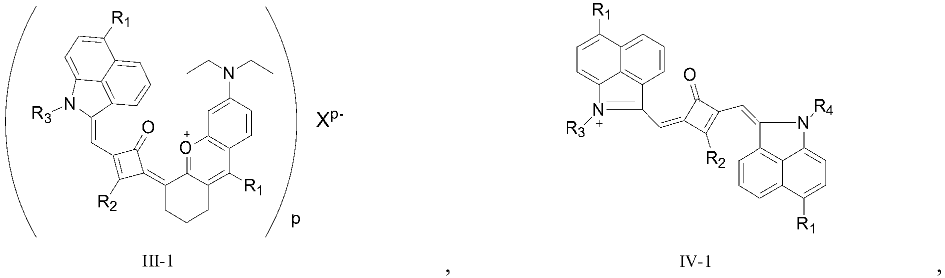

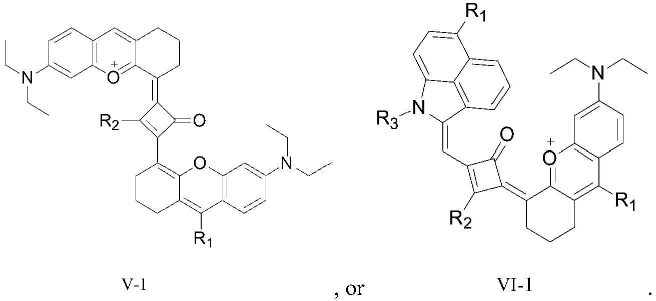

In an embodiment, a substituent of the substituted C₆-C₁₀ aryl or 5 to 10 membered heteroaryl is at least one selected from the group consisting of C₁-C₈ alkyl, C₁-C₈ alkoxy, C₁-C₈ alkylsilyl, hydroxy C₁-C₈ alkyl, amino C₁-C₈ alkyl, mercapto C₁-C₈ alkyl, halo C₁-C₈ alkyl, and carboxy C₁-C₆ alkyl.

In an embodiment, m, n, and p are each independently any integer selected from 1 to 4.

In an embodiment, X⁻ is each independently selected from I⁻, Br⁻, BF₄⁻, or ClO₄⁻.

In an embodiment, the squaric acid compound has any one of following structures:

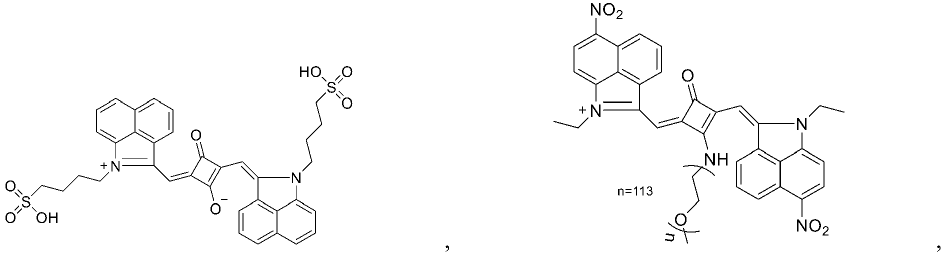

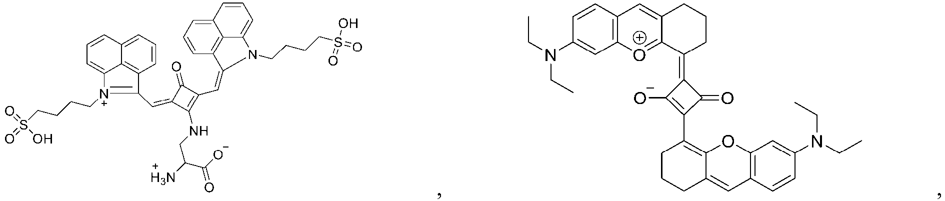

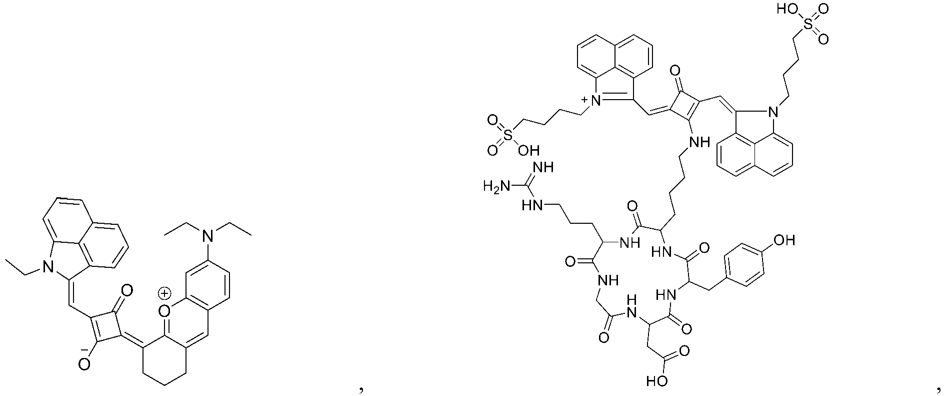

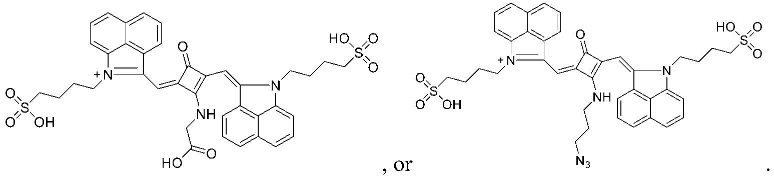

The present application further provides methods for preparing the above-described squaric acid compounds, the technical solutions are as follows:
(1) A method for preparing the squaric acid compound represented by formula I includes the following steps:
   subjecting compound 1 and compound a-1 to a nucleophilic substitution reaction to obtain compound 2-1, the compound a-1 being halogenated R₃, and subjecting the compound 1 and compound a-2 to a nucleophilic substitution reaction to obtain compound 2-2, the compound a-2 being halogenated R₄;
   subjecting the compound 2-1 and compound b to a Grignard reaction to obtain compound 3-1, and subjecting the compound 2-2 and the compound b to a Grignard reaction to obtain compound 3-2, the compound b being a Grignard reagent;
   subjecting the compound 3-1, the compound 3-2, and compound c to a condensation reaction to obtain compound 4 (i.e., compound IV), the compound c being squaric acid;
   reacting compound 4 with compound d-1 to obtain the compound represented by formula I, the compound d-1 containing R₂; 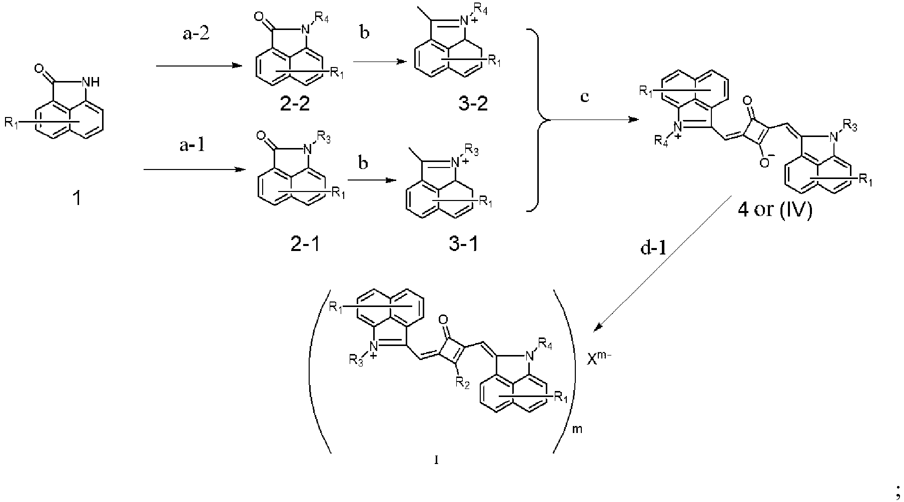
   wherein R₁, R₂, R₃, and R₄ are defined as above.

In an embodiment, the compound a-1 is Br-R₃.

In an embodiment, a molar ratio of the compound 1 to the compound a-1 is in a range from 1:1 to 1:1.5.

In an embodiment, the compound a-2 is Br-R₄.

In an embodiment, a molar ratio of the compound 1 to the compound a-2 is in a range from 1:1 to 1:1.5.

In an embodiment, the compound b is methylmagnesium chloride.

In an embodiment, a molar ratio of the compound 2-1 to the compound b is in a range from 1:1 to 1:1.5.

In an embodiment, a molar ratio of the compound 2-2 to the compound b is in a range from 1:1 to 1:1.5.

In an embodiment, a molar ratio of the compound 3-1 to the compound c is in a range from 1:1 to 3:1.

In an embodiment, a molar ratio of the compound 3-2 to the compound c is in a range from 1:1 to 3:1.

In an embodiment, a molar ratio of the compound 4 to the compound d-1 is in a range from 1:1 to 1:1.5.

(2) A method for preparing the squaric acid compound represented by formula II includes the following steps:
subjecting compound 5 and compound c to a condensation reaction to obtain compound 6 (i.e., compound V), the compound c being squaric acid;
reacting the compound 6 with compound d-2 to obtain the compound represented by formula II, the compound d-2 containing R₂; 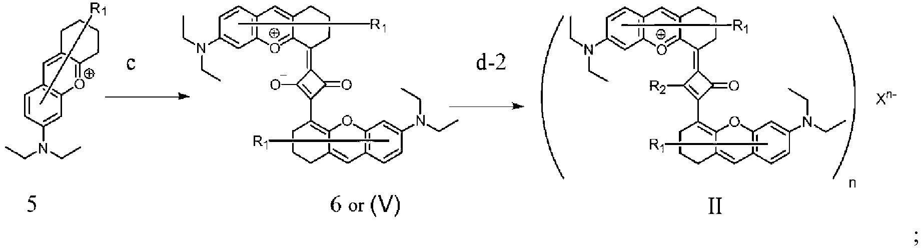
wherein R₁, R₂, R₃, and R₄ are defined as above.

In an embodiment, a molar ratio of the compound 5 to the compound c is in a range from 2:1 to 3:1.

In an embodiment, a molar ratio of the compound 6 to the compound d-2 is in a range from 1:1 to 1:1.5;
(3) A method for preparing the squaric acid compound represented by formula III includes the following steps:
subjecting compound 3-1 and compound e to a substitution reaction to obtain compound 7, the compound e being diethyl squarate;
subjecting the compound 7 and compound f to a hydrolysis reaction to obtain compound 8, the compound f being an alkali;
subjecting the compound 8 and compound 5 to a condensation reaction to obtain compound 9 (i.e., compound VI);
reacting the compound 9 with compound d-3 to obtain the compound represented by formula III, the compound d-3 containing R₂; 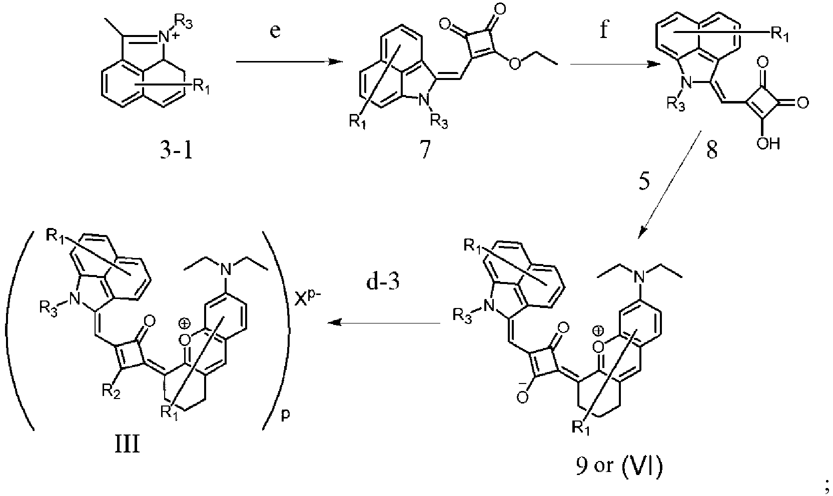
wherein R₁, R₂, R₃, and R₄ are defined as above.

In an embodiment, a molar ratio of the compound 3-1 to the compound e is in a range from 1:1 to 1.5:1;
In an embodiment, a molar ratio of the compound 8 to the compound 5 is in a range from 1:1 to 1:1.5;
In an embodiment, a molar ratio of the compound 9 to the compound d-3 is in a range from 1:1 to 1:1.5.

In another aspect, a fluorescent probe is provided, which includes the squaric acid compound or the pharmaceutically acceptable salt or deuterated compound thereof as described above.

In still another aspect, an NIR-II contrast agent is provided, which includes the squaric acid compound or the pharmaceutically acceptable salt or deuterated compound thereof as described above.

In yet another aspect, a method for detecting early liver injury is provided, which includes imaging a subject in need thereof using the squaric acid compound or the pharmaceutically acceptable salt or deuterated compound thereof as described above, the fluorescent probe as described above, or the NIR-II contrast agent as described above.

The present application has at least the following beneficial effects:
The squaric acid compounds provided by the present application are a class of donor-π-acceptor (D-π-A) type fluorescent molecules emitting near-infrared light, which are developed by taking squaric acid as a central ring and varying the donor and acceptor. The squaric acid compounds exhibit good photostability, low molecular weights, large Stokes shifts, and high molar absorption coefficients, and also possess certain photothermal effects, and therefore they are highly suitable for use as fluorescent probes, particularly near-infrared fluorescent probes. Furthermore, they can be used to prepare NIR-II contrast agents for *in vivo* imaging applications, such as *in vivo* metabolism studies in small animals and lymphangiography.

The preparation method of the squaric acid compound of the present application is simple, and the raw materials are readily available at low cost. The preparation method is very suitable for scale-up production and has broad application prospects.

### BRIEF DESCRIPTION OF THE DRAWINGS

In order to illustrate the technical solutions in the embodiments of the present application and the conventional technology more clearly, the drawings used in the embodiments or the conventional technology will be described briefly. Apparently, the following described drawings are merely for the embodiments of the present application, and other drawings can be derived by those of ordinary skill in the art without any creative effort.

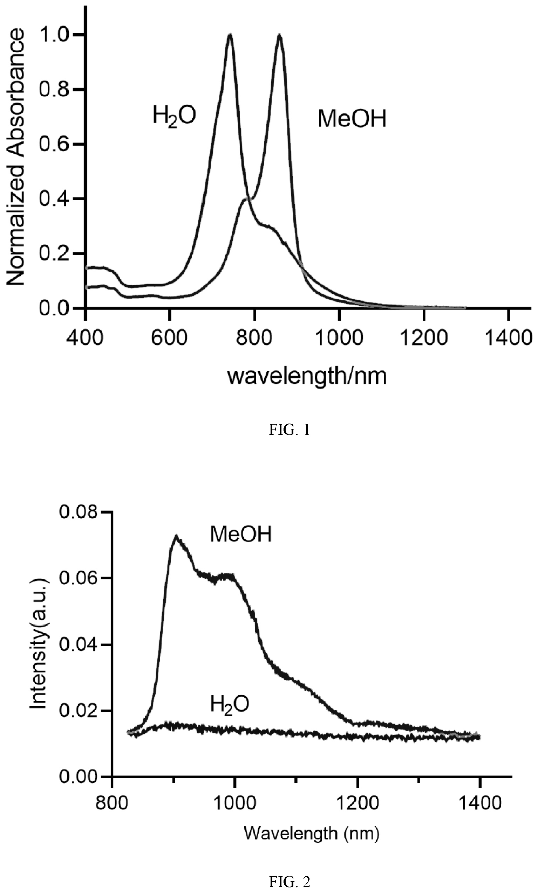

FIG. 1 shows ultraviolet absorption spectra of compound Iₐ.
FIG. 2 shows fluorescence emission spectra of compound Iₐ.
FIG. 3 shows metabolic profile of compound Iₐ in a normal mouse.
FIG. 4 shows metabolic profile of compound Iₐ in a normal mouse and in a carbon tetrachloride-induced liver injury model mouse.
FIG. 5 shows *in vivo* fluorescence images obtained 30 minutes after injection of compound Iₐ into a normal mouse and a carbon tetrachloride-induced liver injury model mouse.
FIG. 6 shows qPCR results from primary hepatocytes extracted from carbon tetrachloride-induced liver injury model mice.
FIG. 7 shows metabolic profile of compound Iₐ in a Mate 1 transporter inhibitor model mouse.
FIG. 8 shows photostability test results of compound Iₐ and currently clinically used indocyanine green (ICG).

### DETAILED DESCRIPTION

The technical solutions of the embodiments of the present application will be described more clearly and comprehensively below in conjunction with the accompanying drawings for the embodiments of the present application. Apparently, the embodiments described herein are only part of, not all of the embodiments of the present application. Based on the embodiments of the present application, other embodiments obtained by those of ordinary skill in the art without creative efforts shall fall within the protection scope of the present application.

Unless defined otherwise, all technical and scientific terms used herein have the same meaning as commonly understood by those skilled in the art of the present application. The terms used in the specification of the present application are for the purpose of describing exemplary examples only and are not intended to limit the present application.

The terms "include", "have", and "contain" used in the present application indicate non-exclusive inclusion. Unless explicit limitation terms such as "only" and "consist of..." are used, another component can also be further added.

In the present application, "further", "furthermore", "specially", etc., are for descriptive purposes, indicating the differences in content, but cannot be understood as a limitation on the protection scope of the present application.

In the present application, "at least one" means one or more, such as one, two, or more than two. "Multiple" or "a few" means at least two, such as two, three, etc. "Multiple layers" means at least two layers, such as two layers, three layers, etc., unless otherwise clearly and specifically limited. In the description of the present application, "several" means at least one, such as one, two, etc., unless otherwise clearly and specifically limited.

In the present application, when a numerical range is mentioned, the range is considered continuous and includes the minimum and maximum values, as well as every value therebetween. Furthermore, when the numerical range refers to integers therein, it includes every integer between the minimum and maximum values. When multiple numerical ranges are provided to describe a feature or characteristic, these numerical ranges can be combined. In other words, unless otherwise indicated, the numerical ranges disclosed in the present application should be understood to encompass any and all subranges included therein.

Unless otherwise specified, all steps in the present application can be carried out sequentially or randomly. For example, when a method includes steps (a) and (b), it means that the method can include steps (a) and (b) performed sequentially, or steps (b) and (a) performed sequentially. For example, when it is mentioned that the method can further include step (c), it means that step (c) can be added to the method in any order, for example, the method can include steps (a), (b) and (c), or steps (a), (c) and (b), or steps (c), (a) and (b), etc.

Unless mentioned otherwise, a term in singular form can include plural form, and should not be construed as being one in quantity.

In the present application, the term "above" or "below" is understood to include the referenced number. For example, "below 1" includes 1.

In the present application, when a temperature parameter is mentioned, unless otherwise specifically stated, not only a thermostatic process but also a variation within a certain temperature interval is allowed. It should be understood that, the thermostatic process allows for temperature fluctuations within the accuracy range of the instrument, such as fluctuation within the range of ±5 °C, ±4 °C, ±3 °C, ±2 °C and ±1 °C.

In the present application, the number of atoms described by a numerical range includes the two integer endpoints of the numerical range and every integer between the two endpoints. For example, "C1 to C10 alkyl" means an alkyl having 1, 2, 3, 4, 5, 6, 7, 8, 9, or 10 carbon atoms.

In the present application, in 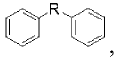 when R is selected from a single bond, 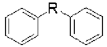 represents 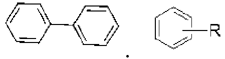 indicates that the linking site where the substituent R is connected to the benzene ring is not limited.

In the present application, 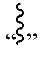 represents a linking site.

In the present application, "halogen" or "halo" refers to -F, -Cl, -Br, or -I.

In the present application, the term "alkyl" refers to a monovalent residue derived from a saturated hydrocarbon containing a primary (normal) carbon atom, or a secondary carbon atom, or a tertiary carbon atom, or a quaternary carbon atom, or any combination thereof, by loss of one hydrogen atom. Phrases containing this term, for example, "C1 to C10 alkyl" refer to an alkyl group containing 1 to 10 carbon atoms, and at each occurrence may independently be C1 alkyl, C2 alkyl, C3 alkyl, C4 alkyl, C5 alkyl, C6 alkyl, C7 alkyl, C8 alkyl, C9 alkyl, or C10 alkyl. Suitable examples include, but are not limited to: methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂, 3,3-dimethyl-2-butyl (-CH(CH₃)C(CH₃)₃), and octyl (-(CH₂)₇CH₃).

In the present application, the term "haloalkyl" refers to an alkyl substituted with one or more halogen atoms including chlorine, fluorine, bromine, or iodine. Polyhaloalkyl has the same or mixed halogen atoms. "Perhaloalkyl" refers to an alkyl in which every hydrogen atom is replaced by a halogen atom. A haloalkyl in which a specific carbon atom is "completely halogenated" means that all hydrogen atoms attached to that carbon are replaced by halogen atoms. Representative mono-, di-, or tri-haloalkyl includes: chloromethyl, chloroethyl, bromomethyl, bromoethyl, iodomethyl, iodoethyl, chloropropyl, bromopropyl, iodopropyl, 1,1-dichloromethyl, 1,1-dibromomethyl, 1,1-dichloropropyl, 1,2-dibromopropyl, 2,3-dibromopropyl, 1-chloro-2-bromoethyl, 2-chloro-3-bromopropyl, trifluoromethyl, trichloromethyl, etc.

In the present application, the term "cycloalkyl" refers to a non-aromatic hydrocarbon containing ring carbon atoms, which can be monocyclic alkyl, spirocycloalkyl, or bridged cycloalkyl. Phrases containing this term, for example, "C3 to C10 cycloalkyl", refer to a cycloalkyl containing 3 to 10 carbon atoms, and at each occurrence can independently be C3 cycloalkyl, C4 cycloalkyl, C5 cycloalkyl, C6 cycloalkyl, C7 cycloalkyl, C8 cycloalkyl, C9 cycloalkyl, or C10 cycloalkyl. Suitable examples include, but are not limited to: cyclopropyl, cyclobutyl, cyclopentyl, cyclohexyl, and cycloheptyl. In addition, "cycloalkyl" can also contain one or more double bonds, and representative examples of cycloalkyl containing a double bond include cyclopentenyl, cyclohexenyl, cyclohexadienyl, and cyclobutadienyl.

In the present application, the term "number of ring atoms" refers to the number of atoms that constitute the ring itself in a structural compound (e.g., monocyclic compound, fused ring compound, bridged compound, carbocyclic compound, or heterocyclic compound) formed by atoms bonded together into a ring. When the ring is substituted with a substituent, the atoms contained in the substituent are not included in the ring atoms. The same applies to the "number of ring atoms" described below unless otherwise specified. For example, the number of ring atoms of a benzene ring is 6, that of a naphthalene ring is 10, and that of a biphenyl is 12.

In the present application, the term "aryl, aryl group or aromatic group" refers to a hydrocarbon group containing at least one aromatic ring, such as benzene, naphthalene, anthracene, fluoranthene, phenanthrene, benzophenanthrene, acenaphthene, fluorene, biphenyl, terphenyl, and derivatives of the above aryls.

In the present application, the term "arylene" refers to an aromatic hydrocarbon group derived from an aromatic ring compound by removal of two hydrogen atoms, which can be monocyclic arylene, fused-ring arylene, or polycyclic arylene, wherein at least one ring in the polycyclic arylene is an aromatic ring. For example, "C6 to C10 arylene" refers to an arylene containing 6 to 10 carbon atoms, and at each occurrence can independently be a C6 arylene, C7 arylene, C8 arylene, C9 arylene, or C10 arylene. Suitable examples include, but are not limited to: phenylene, biphenylene, naphthylene, anthrylene, phenanthrylene, perylenylene, triphenylenylene, and derivatives thereof.

In the present application, the term "cycloalkylene" refers to a hydrocarbon group having two monovalent radical centers, derived from a cycloalkyl by removal of two hydrogen atoms, which can be monocyclic cycloalkylene, spirocycloalkylene, or bridged cycloalkylene. For example, "C3 to C10 cycloalkylene" refers to a cycloalkylene containing 3 to 9 carbon atoms, and at each occurrence can independently be C3 cycloalkylene, C4 cycloalkylene, C5 cycloalkylene, C6 cycloalkylene, C7 cycloalkylene, C8 cycloalkylene, or C9 cycloalkylene. Suitable examples include, but are not limited to: cyclopropylene, cyclobutylene, cyclopentylene, cyclohexylene, and cycloheptylene. In addition, "cycloalkylene" can also contain one or more double bonds, and representative examples of cycloalkylene containing a double bond include cyclopentenylene, cyclohexenylene, cyclohexadienylene, and cyclobutadienylene.

In the present application, "A and B are each independently selected from x, y, or z" means that A and B are independent events, and the occurrence of event A does not affect the occurrence of event B. Therefore, when A is selected from x, B can be any one selected from x, y or z; when A is selected from y, B can be any one selected from x, y or z; and when A is selected from z, B can be any one selected from x, y or z.

In the present application, "substitute" means that a hydrogen atom in the substituted group is replaced by a substituent.

In the present application, "substituted or unsubstituted" means that the defined group may or may not be substituted. When the defined group is substituted, it should be understood as optionally substituted with a group acceptable in the art, including but not limited to: linear alkyl having 1 to 20 carbon atoms, branched or cyclic alkyl having 3 to 20 carbon atoms, heterocyclyl having 3 to 20 ring atoms, aryl having 5 to 20 ring atoms, heteroaryl having 5 to 20 ring atoms, silyl, carbonyl, alkoxycarbonyl, aryloxycarbonyl, carbamoyl, haloformyl, formyl, -NRR', cyano, isocyano, isocyanate, thiocyanate, isothiocyanate, hydroxy, trifluoromethyl, nitro, or halogen. The above groups can be further substituted with a group acceptable in the art, with the selected group including but are not limited to: linear alkyl having 1 to 20 carbon atoms, branched or cyclic alkyl having 3 to 20 carbon atoms, heterocyclyl having 3 to 20 ring atoms, aryl having 5 to 10 ring atoms, heteroaryl having 5 to 10 ring atoms, -NRR', cyano, hydroxy, trifluoromethyl, nitro, or halogen. It should be understood that R and R' in -NRR' are each independently substituted with a group acceptable in the art, including but not limited to H, linear alkyl having 1 to 6 carbon atoms, branched or cyclic alkyl having 3 to 8 carbon atoms, heterocyclyl having 3 to 8 ring atoms, aryl containing 5 to 10 ring atoms, or heteroaryl containing 5 to 10 ring atoms. In addition, the linear alkyl having 1 to 6 carbon atoms, branched or cyclic alkyl having 3 to 8 carbon atoms, heterocyclyl having 3 to 8 ring atoms, aryl having 5 to 10 ring atoms, or heteroaryl having 5 to 10 ring atoms are optionally further substituted, including but not limited to the following substituents: linear alkyl having 1 to 6 carbon atoms, branched or cyclic alkyl having 3 to 8 carbon atoms, aryl having 5 to 10 ring atoms (phenyl or naphthyl in an embodiment), or heteroaryl having 5 to 10 ring atoms.

Inorganic nanomaterials have attracted great interest due to their excellent optical properties. However, inorganic materials are metabolized slowly *in vivo* and remain in the liver and spleen for a long time. The long-term biosafety concerns limit their further clinical translation. Compared with inorganic nanomaterials, small-molecule organic dyes have gained increasing attention from researchers, owing to their advantages including well-defined structures, low molecular weights, rapid metabolism, and safety. Researchers have attempted to extend the emission wavelengths of small-molecule dyes into the near-infrared region by optimizing structural design and synthetic routes. These diverse NIR dye structures enrich the NIR fluorescent probe library through the rational design of the main chain and substituents of the dye structures. Many organic dyes, such as cyanine dyes, D-A-D type dyes, BODIPY type dyes, and porphyrin type dyes, exhibit favorable water solubility, quantum yield, and molar absorption coefficients in biological systems and tissues. Nevertheless, currently only cyanine dyes and D-A-D type dyes have been widely explored as NIR-II fluorescent probes. However, cyanine dyes suffer from poor photostability, inferior chemical stability, low photothermal conversion efficiency, and small Stokes shifts, and the fact that most developed dyes are confined to NIR-I. D-A-D type dyes show drawbacks such as high molecular weights, small molar absorption coefficients, or slow *in vivo* metabolic rates.

The liver performs highly complex and vital physiological functions. As the main metabolic organ in the human body, it is closely linked to various complex biological processes including drug metabolism, excretion, bile secretion, phagocytosis, and the body's immunity. Liver diseases exhibit high incidence and mortality rates in the world. In particular, acute and chronic liver injuries, as well as drug-induced and alcoholic liver injuries seriously threaten human life and health. Moreover, as new drugs continue to enter the market, drug-induced hepatotoxicity will become a major clinical challenge. However, routinely used biomarkers, such as serum alanine aminotransferase (ALT) and aspartate aminotransferase (AST), are insensitive to the detection of early-stage liver function injury, and manifestations of muscular and renal injury in the body may also lead to false positives in diagnosis. In addition, as the secretion and accumulation of ALT and AST, by the time these two indicators rise to detectable levels for monitoring, the liver disease has already progressed to a very severe stage. At this point, the window for timely treatment is missed, and the condition will further deteriorate and threaten life. In the terminal stage of the disease, ALT levels may even decrease instead. Therefore, ALT cannot be regarded as a reliable predictive indicator. Histopathological examination, namely liver biopsy, is currently considered the gold standard for liver disease diagnosis, staging, and grading. However, this technique is invasive and leads to poor patient compliance. In addition, complications may occur at any time during the liver tissue sampling procedure, and the collected samples from partial liver tissue often fail to reflect the actual state of the entire liver, which may lead to a misjudgment of liver injury progression. Early detection of liver function injury is conducive to timely liver-protective intervention. Therefore, developing a reliable method for monitoring liver injury will greatly help ensure drug safety and improve treatment efficiency. Although the currently reported probes possess prominent advantages such as high selectivity and real-time feedback, there are relatively few NIR-II small-molecule fluorescent probes available for the early monitoring of drug-induced liver injury. The FDA-approved fluorescent probe ICG for clinical use is mainly applied to intraoperative navigation for liver resection and preoperative and postoperative liver function assessment, with relatively few studies on its use for monitoring acute liver injury. Furthermore, ICG's inherent photophysical instability limits its application in biological imaging.

Some squaric acid dyes have been reported so far. Squaric acid dyes have the advantages of high molar absorption coefficients (>10⁵ M⁻¹cm⁻¹), excellent photostability, easy, and tunable wavelengths. However, most of the reported squaric acid dyes have emission spectra located in the NIR-I region, with limited penetration depth and high tissue background, adversely affecting the reliability of experimental results. Moreover, the reported dyes are basically lipophilic, with poor water solubility and low bioavailability.

The present application provides a squaric acid compound or a pharmaceutically acceptable salt or deuterated compound thereof. These squaric acid compounds or their pharmaceutically acceptable salts or deuterated compounds emit near-infrared light and have the characteristics of good photostability, low molecular weights, large Stokes shifts, and high molar absorption coefficients, and also possess good water solubility and high bioavailability. Therefore, they can be used for fluorescent probes and for preparing contrast agents, particularly near-infrared II contrast agents.

The technical solutions are as follows:
A squaric acid compound or a pharmaceutically acceptable salt or deuterated compound thereof is represented by a general structural formula I, II, III, IV, V, or VI: 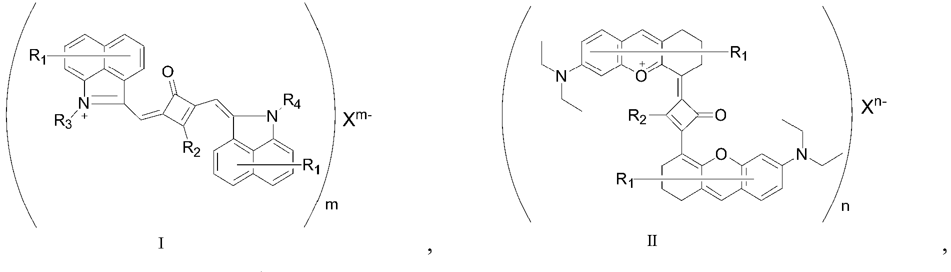 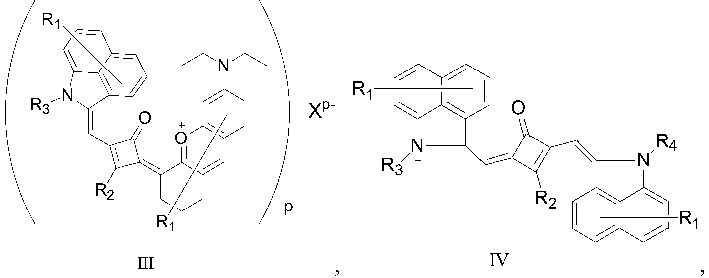 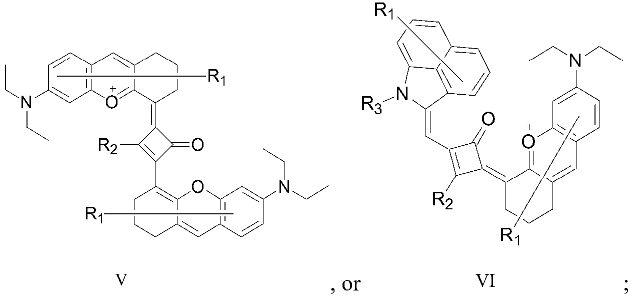
in which, X^{m-}, Xⁿ⁻, and X^{p-} are each independently an anion,
m, n, and p are each independently any integer selected from 1 to 10;
R₁ and R_{1'} are each independently at least one selected from the group consisting of H, C₁-C₁₂ alkyl, C₁-C₁₂ alkoxy, C₁-C₈ alkylsilyl, hydroxy C₁-C₈ alkyl, amino C₁-C₈ alkyl, formyl C₁-C₈ alkyl, mercapto C₁-C₈ alkyl, halo C₁-C₈ alkyl, acyloxy C₁-C₈ alkyl, amino, halogen, nitro, carboxy C₁-C₆ alkyl, substituted or unsubstituted C₆-C₁₀ aryl or 5 to 10 membered heteroaryl, -(CH₂)n₁-COOCH₂CH₂Si(CH₃)₃, -(CH₂)n₂-(CH₂CH₂O)n₃-R, and -(CH₂)n₂-(OCH₂CH₂)n₃-R;
R₂ is at least one selected from the group consisting of hydroxy, C₁-C₁₂ alkoxy, C₁-C₈ alkylsilyl, hydroxy C₁-C₈ alkyl, carboxy, amino, -NR₅R₆, mercapto, -SR₇, and malononitrile;
R₃, R₄, R₅, R₆, and R₇ are each independently at least one selected from the group consisting of H, C₁-C₈ alkyl, hydroxy, amino, carboxy, C₁-C₁₂ alkyl, C₁-C₁₂ alkoxy, C₁-C₈ alkylsilyl, hydroxy C₁-C₈ alkyl, amino C₁-C₈ alkyl, aldehyde C₁-C₈ alkyl, mercapto C₁-C₈ alkyl, halo C₁-C₈ alkyl, acyloxy C₁-C₈ alkyl, amino, halogen, sulfo, carboxy C₁-C₆ alkyl, substituted or unsubstituted C₆-C₁₀ aryl or 5 to 10 membered heteroaryl, -(CH₂)n₁-COOCH₂CH₂Si(CH₃)₃, -(CH₂)n₂-(CH₂CH₂O)n₃-R, and -(CH₂)n₂-(OCH₂CH₂)n₃-R;
n1 is any integer from 0 to 10, n2 is any integer from 0 to 10, and n3 is any integer from 1 to 500;
R is at least one selected from the group consisting of H, C₁-C₈ alkyl, hydroxy, amino, carboxy, sulfo, halogen, mercapto, 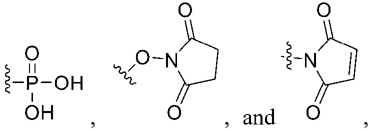 where 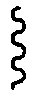 represents a linking site.

In an embodiment, the squaric acid compound is represented by a general structural formula I-1, II-1, III-1, IV-1, V-1, or VI-1:

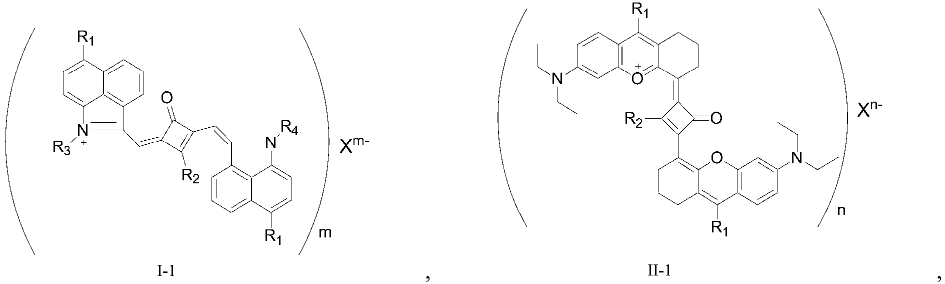

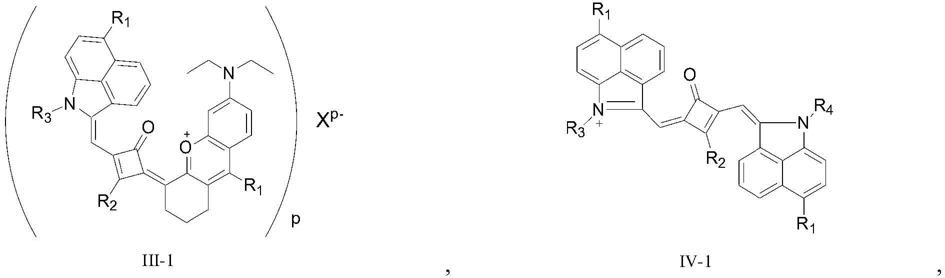

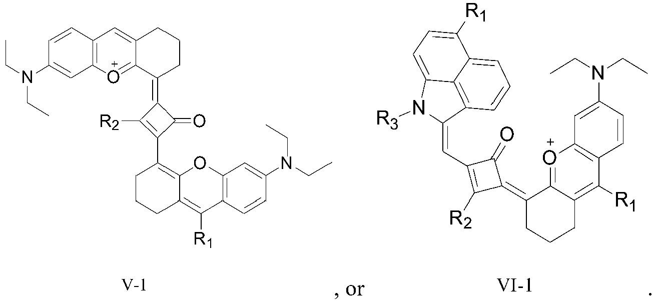

In an embodiment, each R₁ is independently selected from H, nitro, methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃).

In an embodiment, each R₁ is independently selected from hydroxy C₁-C₈ alkyl. In an embodiment, each R₁ is hydroxy C₁-C₄ alkyl.

In an embodiment, each R₁ is independently selected from amino C₁-C₈ alkyl. In an embodiment, each R₁ is amino C₁-C₄ alkyl.

In an embodiment, in R₁, a substituent of the substituted C₆-C₁₀ aryl or 5 to 10 membered heteroaryl is at least one selected from the group consisting of C₁-C₈ alkyl, C₁-C₈ alkoxy, C₁-C₈ alkylsilyl, hydroxy C₁-C₈ alkyl, amino C₁-C₈ alkyl, mercapto C₁-C₈ alkyl, halo C₁-C₈ alkyl, and carboxy C₁-C₆ alkyl.

In an embodiment, R₃ and R₄ are each independently selected from H, methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃).

In an embodiment, R₃ and R₄ are each independently selected from hydroxy C₁-C₈ alkyl. In an embodiment, R₃ and R₄ are each hydroxy C₁-C₄ alkyl.

In an embodiment, R₃ and R₄ are each independently selected from amino C₁-C₈ alkyl. In an embodiment, R₃ and R₄ are each amino C₁-C₄ alkyl.

In an embodiment, in R₃ and R₄, a substituent of the substituted C₆-C₁₀ aryl or 5 to 10 membered heteroaryl is at least one selected from the group consisting of C₁-C₈ alkyl, C₁-C₈ alkoxy, C₁-C₈ alkylsilyl, hydroxy C₁-C₈ alkyl, amino C₁-C₈ alkyl, mercapto C₁-C₈ alkyl, halo C₁-C₈ alkyl, and carboxy C₁-C₆ alkyl.

In the present application, R₂ is selected from the group consisting of hydroxy, O⁻, C₁-C₁₂ alkoxy, C₁-C₈ alkylsilyl, hydroxy C₁-C₈ alkyl, carboxy, amino, -NR₅R₆, mercapto, -SR₇, and malononitrile.

In an embodiment, R₅, R₆, and R₇ are each independently selected from H, methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), octyl (-(CH₂)₇CH₃), -(CH₂)n₁-COOCH₂CH₂Si(CH₃)₃, -(CH₂)n₂-(CH₂CH₂O)n₃-R, and -(CH₂)n₂-(OCH₂CH₂)n₃-R.

In an embodiment, R₅, R₆, and R₇ are each independently selected from hydroxy C₁-C₈ alkyl. In an embodiment, R₅, R₆, and R₇ are each hydroxy C₁-C₄ alkyl.

In an embodiment, R₅, R₆, and R₇ are each independently selected from amino C₁-C₈ alkyl. In an embodiment, R₅, R₆, and R₇ are each amino C₁-C₄ alkyl.

In an embodiment, in R₅, R₆, and R₇, a substituent of the substituted C₆-C₁₀ aryl or 5 to 10 membered heteroaryl is at least one selected from the group consisting of C₁-C₈ alkyl, C₁-C₈ alkoxy, C₁-C₈ alkylsilyl, hydroxy C₁-C₈ alkyl, amino C₁-C₈ alkyl, mercapto C₁-C₈ alkyl, halo C₁-C₈ alkyl, and carboxy C₁-C₆ alkyl.

It can be understood that, each of X^{m-}, xⁿ⁻, and X^{p-} is an anion, and m, n, and p are each any integer independently selected from 1 to 10, i.e., m, n, and p are each selected from 1, 2, 3, 4, 5, 6, 7, 8, 9, or 10. In an embodiment, m, n, and p are each any integer independently selected from 1 to 4, i.e., 1, 2, 3, or 4. In an embodiment, m, n, and p are each independently selected from 1 or 2. In some other embodiment, each of m, n, and p is 1. X⁻ is each independently selected from I⁻, Br⁻, BF₄⁻, or ClO₄⁻.

It can be understood that, in the present application, n1 is any integer from 0 to 10, i.e., n1 is 0, 1, 2, 3, 4, 5, 6, 7, 8, 9, or 10. In an embodiment, n1 is any integer from 0 to 6.

It can be understood that, in the present application, n2 is any integer from 0 to 10, i.e., n2 is 0, 1, 2, 3, 4, 5, 6, 7, 8, 9, or 10. In an embodiment, n2 is any integer from 0 to 6.

It can be understood that, in the present application, n3 is any integer from 1 to 500, i.e., n3 is 1, 2, 3, 4, 5, 6, 7, 8, 9, 10, 15, 20, 30, 40, 50, 60, 70, 80, 90, 100, 150, 200, 250, 300, 350, 400, 450, or 500. In an embodiment, n3 is any integer from 1 to 300. In another embodiment, n3 is any integer from 1 to 150.

In an embodiment, the squaric acid compound has any one of the following structures:

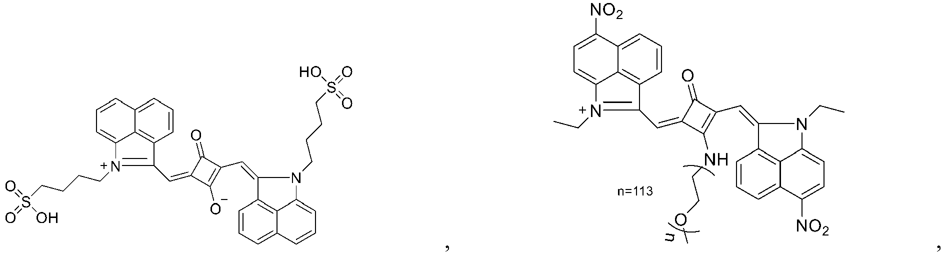

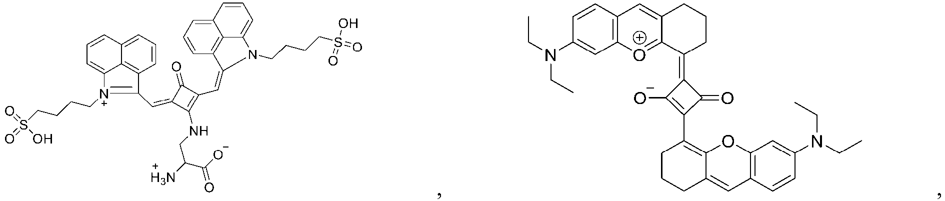

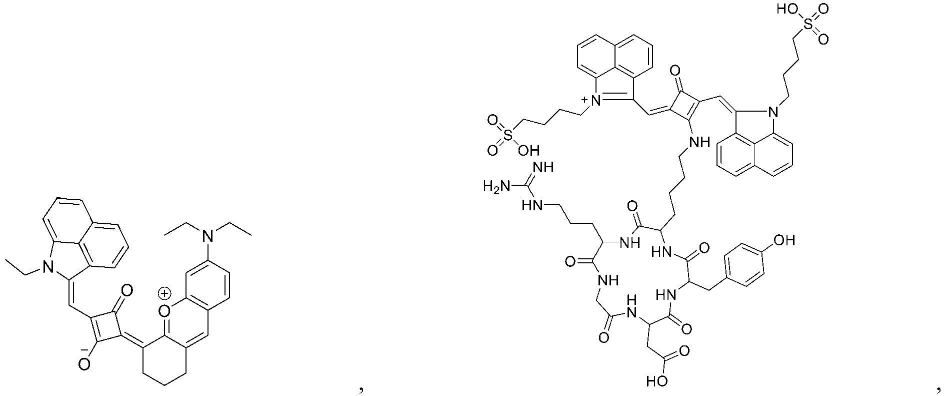

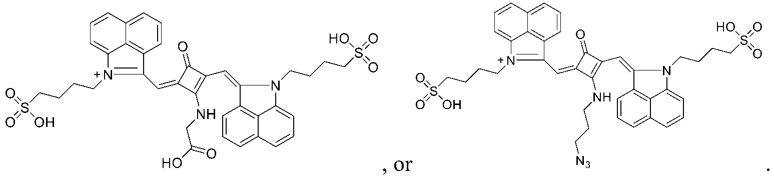

The present application further provides methods for preparing the above-described squaric acid compounds, the technical solutions are as follows:
(1) A method for preparing the squaric acid compound represented by formula I includes the following steps:
   subjecting compound 1 and compound a-1 to a nucleophilic substitution reaction to obtain compound 2-1, the compound a-1 being halogenated R₃, and subjecting the compound 1 and compound a-2 to a nucleophilic substitution reaction to obtain compound 2-2, the compound a-2 being halogenated R₄;
   subjecting the compound 2-1 and compound b to a Grignard reaction to obtain compound 3-1, and subjecting the compound 2-2 and the compound b to a Grignard reaction to obtain compound 3-2, the compound b being a Grignard reagent;
   subjecting the compound 3-1, the compound 3-2, and compound c to a condensation reaction to obtain compound 4 (i.e., compound IV), the compound c being squaric acid;
   reacting compound 4 with compound d-1 to obtain the compound represented by formula I, the compound d-1 containing R₂; 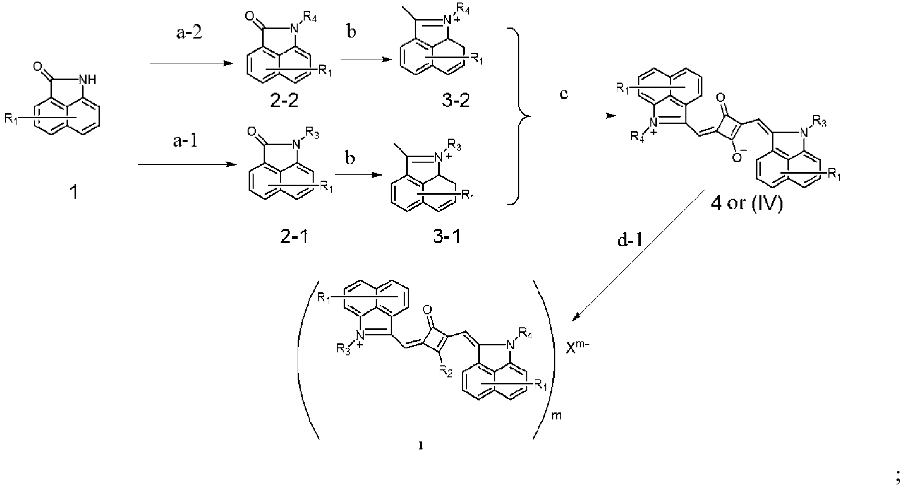 wherein R₁, R₂, R₃, and R₄ are defined as above.

In an embodiment, the compound a-1 is Br-R₃.

In an embodiment, a molar ratio of the compound 1 to the compound a-1 is in a range from 1:1 to 1:1.5.

In an embodiment, the compound a-2 is Br-R₄.

In an embodiment, a molar ratio of the compound 1 to the compound a-2 is in a range from 1:1 to 1:1.5.

In an embodiment, the compound b is methylmagnesium chloride.

In an embodiment, a molar ratio of the compound 2-1 to the compound b is in a range from 1:1 to 1:1.5.

In an embodiment, a molar ratio of the compound 2-2 to the compound b is in a range from 1:1 to 1:1.5.

In an embodiment, a molar ratio of the compound 3-1 to the compound c is in a range from 1:1 to 3:1.

In an embodiment, a molar ratio of the compound 3-2 to the compound c is in a range from 1:1 to 3:1.

In an embodiment, a molar ratio of the compound 4 to the compound d-1 is in a range from 1:1 to 1:1.5.

In an embodiment, a method for preparing the squaric acid compound represented by formula I includes the following steps:

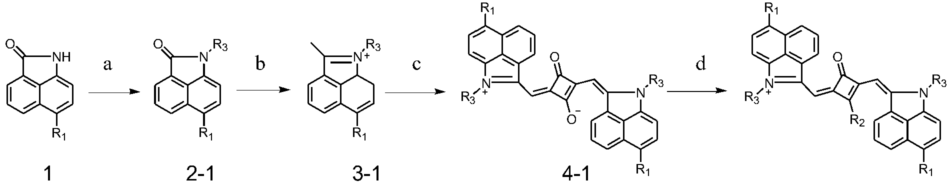

5.91 mmol of compound 1 is dissolved in DMF, and 7.09 mmol of sodium hydride is added portionwise at 0 °C in an ice bath. The mixture is stirred for 10 minutes, and then iodomethane (0.56 mL, 7.09 mmol) is added in an ice bath. The reaction is carried out at room temperature overnight under nitrogen protection. After the reaction is completed, the reaction solution is extracted with ethyl acetate and a saturated aqueous sodium chloride solution. The organic phase is dried over anhydrous sodium sulfate and filtered. The filtrate is adsorbed onto silica gel and subjected to column chromatography to obtain compound 2-1.

5.07 mmol of compound 2-1 is placed in a two-neck flask, dissolved in anhydrous tetrahydrofuran, and the flask is purged with nitrogen. Methylmagnesium chloride (2.03 mL, 6.08 mmol) is added under heating at 60 °C, and the mixture is heated under reflux overnight. After the reaction is completed, the reaction solution is poured into a 2 M hydrochloric acid solution in an ice bath to quench the reaction. Subsequently, a saturated aqueous sodium tetrafluoroborate solution is added, and the mixture is stirred for 30 minutes, during which a solid is precipitated. The green solid is collected by centrifugation, washed with a small amount of water, and dried to obtain compound 3-1.

0.87 mmol of squaric acid and 1.75 mmol of compound 3-1 are charged into a flask. 10 mL of n-butanol and 10 mL of toluene are added as reaction solvents. Toluene is added into the Dean-Stark apparatus up to the side arm, and the flask is purged with nitrogen. The reaction mixture is stirred at 130 °C for 3 to 4 hours. After the reaction is completed, toluene is removed by rotary evaporation under reduced pressure. The resulting solution is filtered, and the solid is washed with anhydrous diethyl ether until the ether is colorless, followed by silica gel column chromatography to obtain compound 4-1.

0.3 mmol of compound 4-1 is charged into a two-neck flask, and the flask is purged with nitrogen. Anhydrous dichloromethane is added to dissolve the compound and the flask is purged with nitrogen again. 0.36 mmol of methyl trifluoromethanesulfonate is added via a syringe, and the reaction is carried out at room temperature overnight. After the reaction is completed, the reaction is quenched with a 5 wt% aqueous sodium bicarbonate solution, followed by extraction with water and dichloromethane. The organic phase is dried over anhydrous sodium sulfate, and the filtrate is adsorbed onto silica gel, and subjected to column chromatography to obtain compound I.

(2) A method for preparing the squaric acid compound represented by formula II includes the following steps:
subjecting compound 5 and compound c to a condensation reaction to obtain compound 6 (i.e., compound V), the compound c being squaric acid;
reacting the compound 6 with compound d-2 to obtain the compound represented by formula II, the compound d-2 containing R₂; 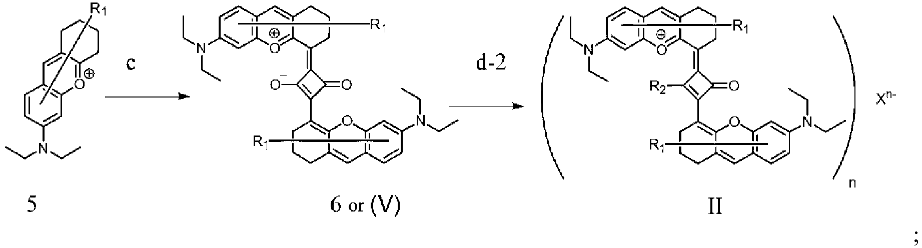
wherein R₁, R₂, R₃, and R₄ are defined as above.

In an embodiment, a molar ratio of the compound 5 to the compound c is in a range from 2:1 to 3:1.

In an embodiment, a molar ratio of the compound 6 to the compound d-2 is in a range from 1:1 to 1:1.5;

In an embodiment, a method for preparing the squaric acid compound represented by formula II includes the following steps:

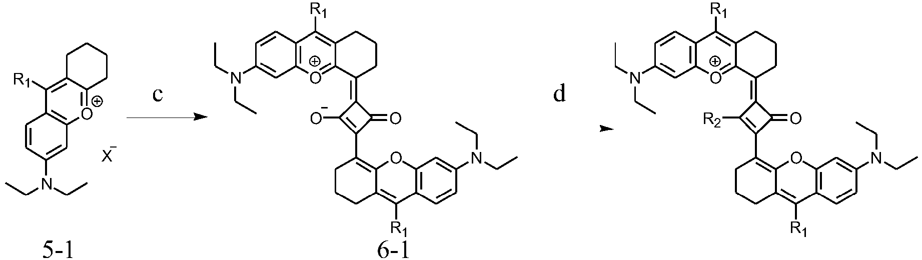

0.87 mmol of squaric acid and 1.75 mmol of compound 5-1 are charged into a flask, 10 mL of n-butanol and 10 mL of toluene are added as reaction solvents. Toluene is added into the Dean-Stark apparatus up to the side arm, and the flask is purged with nitrogen. The reaction mixture is stirred at 130 °C for 3 to 4 hours. After the reaction is completed, toluene is removed by rotary evaporation under reduced pressure. The resulting solution is filtered, and the solid is washed with anhydrous diethyl ether until the ether is colorless, followed by silica gel column chromatography to obtain compound 6-1.

0.3 mmol of compound 6-1 is added to a two-neck flask, and the flask is purged with nitrogen. Anhydrous dichloromethane is added to dissolve the compound and the flask is purged with nitrogen again. 0.36 mmol of methyl trifluoromethanesulfonate is added via a syringe, and the reaction is carried out at room temperature overnight. After the reaction is completed, the reaction is quenched with 5 wt% aqueous sodium bicarbonate solution, followed by extraction with water and dichloromethane. The organic phase is dried over anhydrous sodium sulfate, and the filtrate is adsorbed onto silica gel, and subjected to column chromatography to obtain compound II.

(3) A method for preparing the squaric acid compound represented by formula III includes the following steps:
subjecting compound 3-1 and compound e to a substitution reaction to obtain compound 7, the compound e being diethyl squarate;
subjecting the compound 7 and compound f to a hydrolysis reaction to obtain compound 8, the compound f being an alkali;
subjecting the compound 8 and compound 5 to a condensation reaction to obtain compound 9 (i.e., compound VI);
reacting the compound 9 with compound d-3 to obtain the compound represented by formula III, the compound d-3 containing R₂; 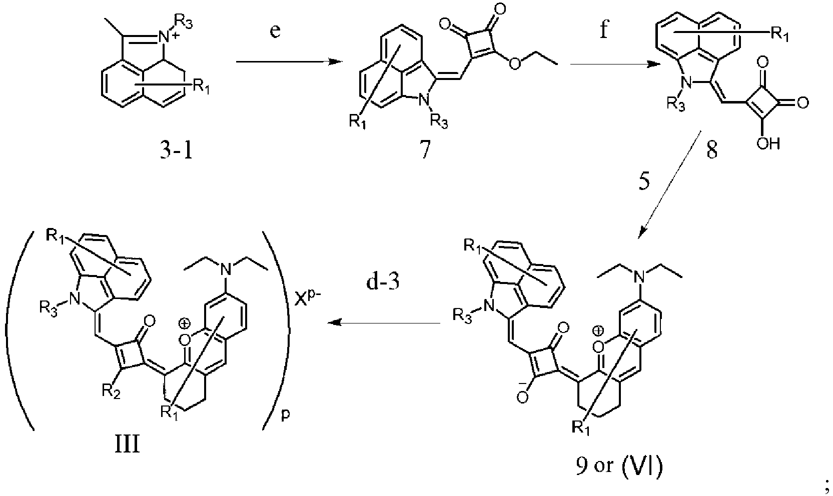
wherein R₁, R₂, R₃, and R₄ are defined as above.

In an embodiment, a molar ratio of the compound 3-1 to the compound e is in a range from 1:1 to 1.5:1;

In an embodiment, a molar ratio of the compound 8 to the compound 5 is in a range from 1:1 to 1:1.5;

In an embodiment, a molar ratio of the compound 9 to the compound d-3 is in a range from 1:1 to 1:1.5.

In an embodiment, a method for preparing the squaric acid compound represented by formula III includes the following steps:

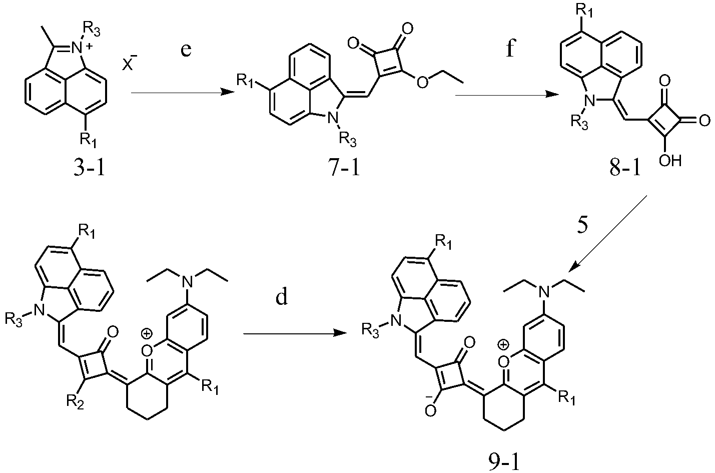

3.98 mmol of compound 3-1, 3.32 mmol of diethyl squarate, and 8.96 mmol of triethylamine are charged into a flask, followed by the addition of ethanol. The flask is purged with nitrogen, and the mixture is heated under reflux at 90 °C overnight. After the reaction solution is cooled to room temperature, the solvent is removed. The residue is adsorbed onto silica gel, and subjected to column chromatography to obtain compound 7.

1.20 mmol of compound 7-1 is charged into a flask, and dissolved in ethanol. A 40% aqueous sodium hydroxide solution is added under reflux and reacted for 2 to 3 hours. After the reaction is completed, the mixture is cooled to room temperature, and a 2 M HCl solution is added to adjust the pH to neutral. The reaction solution is concentrated, and 5 mL of ice-cold ethanol solution is added to precipitate a solid. The solid is collected by filtration to obtain compound 8-1.

1.2 mmol of compound 8-1 and 1.75 mmol of compound 5 are charged into a flask, and 10 mL of n-butanol and 10 mL of toluene are added as reaction solvents. Toluene is added into a Dean-Stark apparatus up to the side arm, and the flask is purged with nitrogen. The reaction solution is stirred at 130 °C for 3 to 4 hours. After the reaction is completed, toluene is removed by rotary evaporation under reduced pressure. The resulting solution is filtered, and the solid is washed with anhydrous diethyl ether until the ether is colorless, followed by silica gel column chromatography to obtain compound 9-1.

0.3 mmol of compound 9-1 is charged into a two-neck flask, and the flask is purged with nitrogen. Anhydrous dichloromethane is added to dissolve the compound and the flask is purged with nitrogen again. 0.36 mmol of methyl trifluoromethanesulfonate is added via a syringe, and the reaction is carried out at room temperature overnight. After the reaction is completed, the reaction is quenched with a 5 wt% aqueous sodium bicarbonate solution, followed by extraction with water and dichloromethane. The organic phase is dried over anhydrous sodium sulfate, and the filtrate is adsorbed onto silica gel, and subjected to column chromatography to obtain compound III.

The present application further provides use of the squaric acid compound as described above, and the technical solutions are as follows:

A fluorescent probe includes the squaric acid compound or the pharmaceutically acceptable salt or deuterated compound thereof as described above.

An NIR-II contrast agent includes the squaric acid compound or the pharmaceutically acceptable salt or deuterated compound thereof as described above.

A method for detecting early liver injury includes imaging a subject in need thereof using the squaric acid compound or the pharmaceutically acceptable salt or deuterated compound thereof as described above, the fluorescent probe as described above, or the NIR-II contrast agent as described above. In this aspect, the present application further provides use of the squaric acid compound or the pharmaceutically acceptable salt or deuterated compound thereof as described above, the fluorescent probe as described above, or the NIR-II contrast agent as described above in monitoring early liver injury in a subject in need thereof.

Specific examples are set forth below to illustrate the present application.

**Table 1: Structures of compounds synthesized in examples**

| No. | Chemical structure | No. | Chemical structure |
|---|---|---|---|
| Ia | 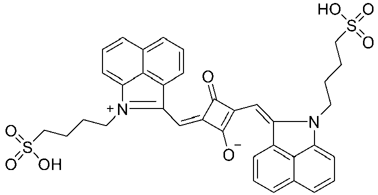 | Ib | 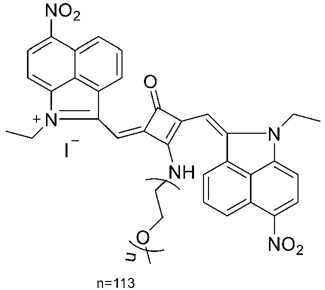 |
| Ic | 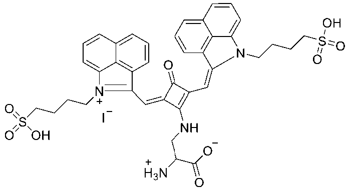 | Id | 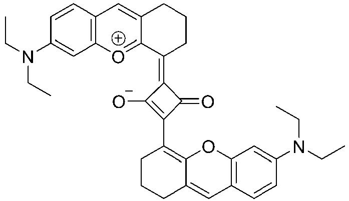 |
| Ie | 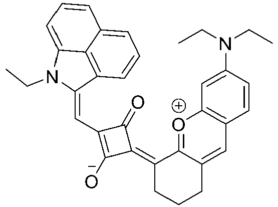 | If | 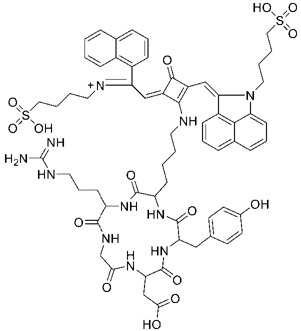 |
| Ig | 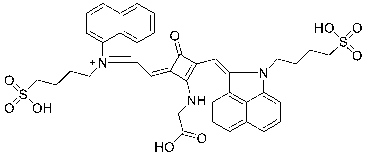 | Ih | 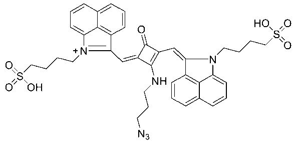 |

### Example 1: Synthesis of compound Ia

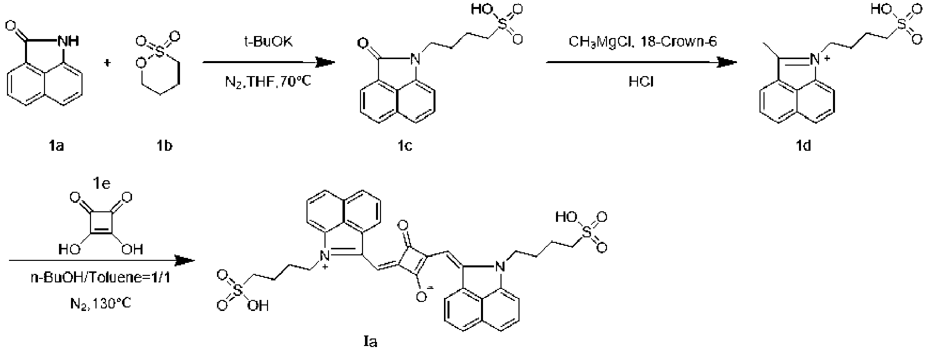

Compound 1a (3 g, 17.73 mmol) and potassium tert-butoxide (1.99 g, 17.73 mmol) were weighed and added to a three-neck flask. Anhydrous THF was added as the solvent to dissolve both compounds. The flask was purged with nitrogen, and the reaction solution was stirred under reflux at 70 °C for 10 minutes. Then compound 1b (2.41 g, 17.73 mmol, 1.81 mL) was added via a syringe, and the reaction was continued for 2 hours. A white precipitate formed during the reaction. The reaction progress was monitored by high-performance liquid chromatography (HPLC). Once compound 1a was completely consumed, the resulting mixture was directly subjected to the next step without workup.

After compound 1a was completely converted to compound 1c, heating was stopped. 18-Crown-6 (4.69 g, 17.73 mmol) was added, and the mixture was stirred for 10 minutes. The flask was purged with nitrogen, and methylmagnesium chloride (4.64 g, 62.1 mmol, 20.70 mL) was then slowly added dropwise via a syringe. The reaction was continued for 2 to 3 hours, with the progress monitored by HPLC. After the reaction was complete, the reaction solution was cooled to room temperature. The reaction solution was poured into a 3 M HCl solution (35.5 mL) in an ice bath and stirred for 20 minutes. Then 30 mL of ethanol was added and stirring was continued for 10 minutes. The resulting suspension was centrifuged by a centrifuge. The solid was washed with acetonitrile, filtered under suction, and dried to obtain 4.1 g of green solid 1d, with a yield of 75.9%.

¹H NMR (400 MHz, D2O) δ 8.48 (d, J=7.3 Hz, 1H), 8.39 (d, J=8.1 Hz, 1H), 8.07 (dd, J=9.6, 7.9 Hz, 2H), 7.84 (t, J=7.7 Hz, 1H), 7.73 (t, J=7.8 Hz, 1H), 4.52 (t, J=7.6 Hz, 2H), 2.89 (t, J=7.5 Hz, 2H), 2.06 (p, J=7.7 Hz, 2H), 1.83 (p, J=7.6 Hz, 2H).

¹³C NMR (126 MHz, D2O) δ 171.2, 138.4, 137.8, 134.1, 130.6, 130.1, 128.9, 128.4, 127.9, 121.8, 120.1, 49.6, 45.9, 27.5, 21.1.

Compound 1e (0.2 g, 1.75 mmol) and compound 1d (1.12 g, 3.51 mmol) were added to a flask, and 10 mL of n-butanol and 10 mL of toluene were added as solvents. Toluene was added to into a Dean-Stark apparatus up to the side arm. The flask was purged with nitrogen. The reaction solution was stirred at 130 °C for 3 to 4 hours. After the reaction was completed, toluene was removed by rotary evaporation under reduced pressure. The resulting solution was filtered, and the solid was washed with anhydrous diethyl ether until the ether became colorless. The resulting solid was dissolved in water and subjected to reverse-phase silica gel column chromatography to obtain compound 1a as a green solid.

¹H NMR (400 MHz, DMSO-d6) δ 9.00 (d, J=7.4 Hz, 2H), 8.11 (d, J=8.0 Hz, 2H), 7.88 (t, J=7.7 Hz, 2H), 7.68 (d, J=8.2 Hz, 2H), 7.60 (t, J=7.7 Hz, 2H), 7.49 (d, J=7.3 Hz, 2H), 6.27 (s, 2H), 4.32 (t, J=7.3 Hz, 4H), 2.57 (t, J=7.5 Hz, 5H), 1.89 (p, J=7.4 Hz, 5H), 1.75 (h, J=7.2, 6.2 Hz, 5H).

¹³C NMR (126 MHz, DMSO) δ 181.6, 175.8, 149.5, 141.1, 130.7, 129.8, 129.6, 129.4, 129.2, 129.1, 124.4, 121.6, 108.7, 91.8, 50.9, 43.2, 27.6, 22.5.

ESI-LR:
Ia: expected M.W: about 685. Found: about 685.

### Example 2: Synthesis of Compound Ib

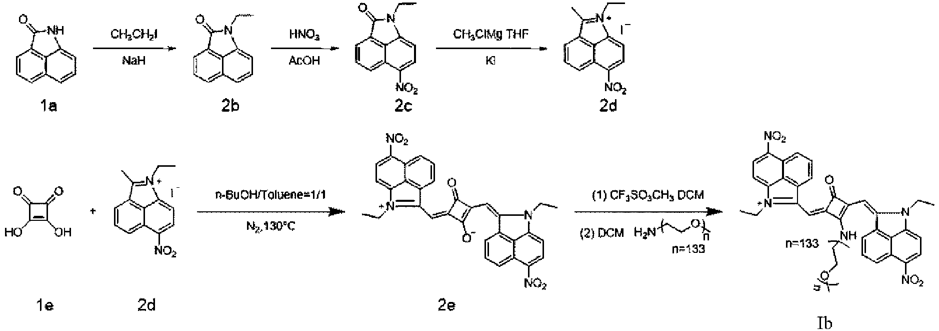

At 0 °C, 0.28 g of sodium hydride was added portionwise to a solution of compound 1a (1 g) in DMF, and the mixture was stirred for 15 minutes. 567 µL of iodoethane was then added, and the reaction was carried out at room temperature for 12 hours. The reaction was monitored by TLC. After the reaction was complete, the reaction solution was poured into saturated brine, and extracted with ethyl acetate three times. The organic layers were combined, dried, and subjected to silica gel column chromatography to obtain 0.8 g of compound 2b, with a yield of 68%.

¹H NMR (500 MHz, Chloroform-*d*) δ 7.93-7.87 (m, 1H), 7.81 (dd, *J*=7.5, 1.6 Hz, 1H), 7.67 (t, *J*=7.5 Hz, 1H), 7.64-7.56 (m, 2H), 6.99 (dd, *J*=6.5, 2.5 Hz, 1H), 4.11 (q, *J*=8.0 Hz, 2H), 1.31-1.24 (m, 3H).

At 0 °C, nitric acid was added to a solution of compound 2b (0.5 g) in acetic acid, and the reaction solution was reacted at 50 °C for 12 hours. The reaction was monitored by TLC. After the reaction was complete, the reaction solution was extracted with water and ethyl acetate, and the organic layer was dried and subjected to silica gel column chromatography to obtain 0.46 g of compound 2c, with a yield of 75%.

¹H NMR (500 MHz, Chloroform-*d*) δ 9.01 (dd, *J*=7.6, 1.5 Hz, 1H), 8.21 (d, *J*=7.5 Hz, 1H), 7.86 (dd, *J*=7.5, 1.5 Hz, 1H), 7.75 (t, *J*=7.4 Hz, 1H), 7.25 (d, *J*=7.5 Hz, 1H), 4.12 (q, *J*=8.0 Hz, 2H), 1.28 (t, *J*=8.0 Hz, 3H).

0.5 g of compound 2c was dissolved in THF, the flask was purged with nitrogen, and 2.5 mL of methylmagnesium chloride was then added. The reaction was carried out at 70 °C for 12 hours. The reaction was monitored by HPLC. After the reaction was complete, the reaction solution was cooled to room temperature, and poured into ice water containing 2.5 mL of HCl. Subsequently, 1 g of KI was added, and the mixture was filtered to obtain 0.4 g of compound 2d, with a yield of 76%.

¹H NMR (500 MHz, Chloroform-*d*) δ 9.02 (dd, *J*=7.6, 1.6 Hz, 1H), 8.37 (d, *J*=7.5 Hz, 1H), 7.83 (t, *J*=7.5 Hz, 1H), 7.67 (dd, *J*=7.5, 1.6 Hz, 1H), 7.53 (d, *J*=7.5 Hz, 1H), 4.80 (q, *J*=8.0 Hz, 2H), 2.88 (s, 2H), 1.53 (t, *J*=8.0 Hz, 3H).

100 mg of compound 1e and 710 mg of compound 2d were weighed, and 10 mL of n-butanol and 10 mL of toluene were added. The flask was purged with nitrogen, and the reaction was carried out at 130 °C for 12 h. The reaction was monitored by TLC. After the reaction was complete, column chromatography was performed to obtain 0.2 g of compound 2e, with a yield of 40%.

¹H NMR (500 MHz, Chloroform-*d*) δ 9.01 (dd, *J*=7.6, 1.6 Hz, 1H), 8.43 (d, *J*=7.5 Hz, 1H), 8.26-8.18 (m, 3H), 7.75-7.66 (m, 3H), 7.65-7.58 (m, 2H), 7.54 (t, *J*=7.5 Hz, 1H), 7.33 (d, *J*=7.5 Hz, 1H), 4.83 (q, *J*=8.0 Hz, 2H), 4.09 (q, *J*=8.0 Hz, 2H), 1.50 (t, *J*=8.0 Hz, 3H), 1.35 (t, *J*=8.0 Hz, 3H).

10 mg of compound 2e was dissolved in THF, 100 µL of methyl trifluoromethanesulfonate was added, and the mixture was stirred and reacted at room temperature for 5 hours. The reaction progress was monitored by HPLC. After the reaction was completed, the solvent was removed by rotary evaporation to give a residue, which was used directly in the next step. The residue was dissolved in anhydrous DMSO, followed by the addition of 3 mg of NH₂-PEG5K, and the reaction was carried out at room temperature for 12 hours. After the reaction was completed, the reaction solution was dialyzed using a dialysis bag, and further purified by C18 reverse-phase column chromatography. The final product was characterized by MALDI-TOF-MS.

MALDI-TOF-MS:
Ib: expected M.W: about 5558. Found: about 5550.

### Example 3: Synthesis of Compound Ic

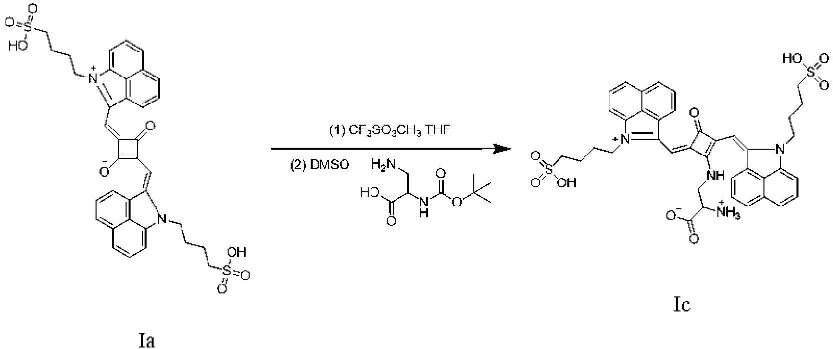

10 mg of compound Ia was dissolved in THF, 100µL of methyl trifluoromethanesulfonate was added, and the mixture was stirred and reacted at room temperature for 5 hours. The reaction progress was monitored by HPLC. After the reaction was completed, the solvent was removed by rotary evaporation to give a residue, which was used directly in the next step. The residue was dissolved in anhydrous DMSO, 3 mg of 3-amino-N-(tert-butoxycarbonyl)alanine was added, and the reaction was carried out at room temperature for 12 hours. After the reaction was completed, the reaction solution was dialyzed using a dialysis bag, viand further purified by C18 reverse-phase column chromatography. The final product was characterized by MALDI-TOF-MS.

¹H NMR (500 MHz, Chloroform-*d*) δ 8.49 (s, 1H), 8.45-8.37 (m, 2H), 8.24 (s, 1H), 8.05-7.97 (m, 3H), 7.80 (dtd, *J*=7.5, 4.6, 4.1, 2.3 Hz, 3H), 7.71 (dt, *J*=7.5, 1.5 Hz, 1H), 7.69-7.62 (m, 3H), 7.55 (dt, *J*=7.5, 1.8 Hz, 2H), 7.50 (td, *J*=7.4, 5.0 Hz, 2H), 7.38 (t, *J*=7.5 Hz, 1H), 7.15 (dd, *J*=7.5, 1.7 Hz, 1H), 4.32-4.23 (m, 1H), 4.22-4.04 (m, 3H), 3.96-3.83 (m, 2H), 3.82-3.72 (m, 1H), 3.03-2.91 (m, 2H), 2.94-2.81 (m, 2H), 2.09-2.02 (m, 1H), 2.05-1.98 (m, 2H), 2.02-1.93 (m, 1H), 1.90-1.73 (m, 4H).

MALDI-TOF-MS:
Ic: expected M.W: about 771. Found: about 771.

### Example 4: Synthesis of Compound Id

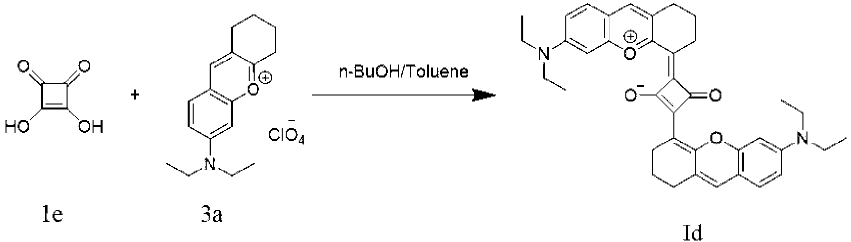

Compound 1e (0.1 g, 1.75 mmol) and compound 3a (0.675 g, 3.51 mmol) were charged into a flask, and 10 mL of n-butanol and 10 mL of toluene were added as solvents. Toluene was added into a Dean-Stark apparatus up to the side arm. The flask was purged with nitrogen. The reaction solution was stirred at 130 °C for 3 to 4 hours. After the reaction was completed, toluene was removed by rotary evaporation under reduced pressure. The resulting solution was filtered, and the solid was washed with anhydrous diethyl ether until the ether became colorless. The resulting solid was dissolved in water and subjected to silica gel column chromatography to obtain 0.2 g of compound Id, with a yield of 38.75%.

¹H NMR (500 MHz, Chloroform-*d*) δ 7.80 (dd, *J*=7.5, 2.3 Hz, 1H), 7.60 (dt, *J*=2.2, 0.9 Hz, 1H), 7.41 (d, *J*=7.5 Hz, 1H), 7.26 (dd, *J*=7.6, 1.6 Hz, 1H), 6.92-6.88 (m, 1H), 6.71 (dd, *J*=11.0, 1.4 Hz, 2H), 6.51 (dd, *J*=7.5, 1.5 Hz, 1H), 3.49 (dq, *J*=10.1, 8.0 Hz, 8H), 3.17 (t, *J*=7.1 Hz, 2H), 2.91-2.83 (m, 2H), 2.87-2.76 (m, 2H), 2.75 (t, *J*=7.1 Hz, 2H), 2.01 (p, *J*=7.1 Hz, 2H), 1.64 (p, *J*=7.1 Hz, 2H), 1.17 (t, *J*=8.0 Hz, 12H).

### Example 5: Synthesis of Compound Ie

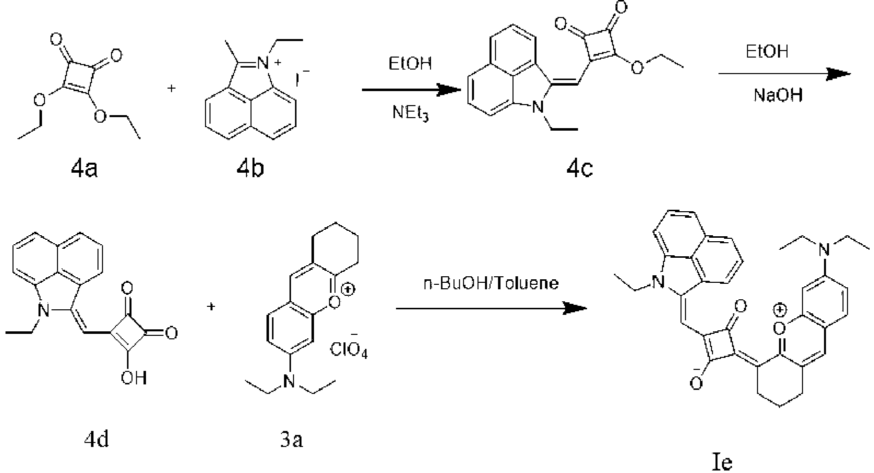

0.5 g of compound 4b was dissolved in 3 mL of ethanol and heated under reflux. 0.3 mL of compound 4a and 0.6 mL of triethylamine were dissolved in 0.3 mL of ethanol, and added to the reaction solution. The reaction was carried out for 12 hours. After the reaction was completed, the reaction solution was cooled to room temperature. The solvent was removed under reduced pressure, and the solid was subjected to silica gel column chromatography to obtain 0.35 g of compound 4c, with a yield 52%.

¹H NMR (500 MHz, Chloroform-*d*) δ 7.84-7.78 (m, 1H), 7.81 (s, 3H), 7.67 (dt, *J*=7.9, 1.7 Hz, 2H), 7.51-7.41 (m, 6H), 6.94 (dd, *J*=7.5, 1.5 Hz, 2H), 4.19 (q, *J*=8.1 Hz, 4H), 4.09 (q, *J*=8.0 Hz, 4H), 1.47 (t, *J*=8.0 Hz, 6H), 1.32 (t, *J*=8.0 Hz, 6H).

0.3 g of compound 4c was dissolved in ethanol and heated under reflux. A 40% aqueous sodium hydroxide solution (0.2 mL) was added, and the reaction was carried out for 2 hours. Subsequently, the solvent was removed, and the residue was subjected to column chromatography to obtain 0.21 g of compound 4d, with a yield of 49%.

¹H NMR (500 MHz, Chloroform-*d*) δ 7.84-7.75 (m, 2H), 7.65-7.60 (m, 2H), 7.51-7.41 (m, 3H), 6.90 (dd, *J*=7.5, 1.6 Hz, 1H), 4.10 (q, *J*=8.0 Hz, 2H), 1.32 (t, *J*=8.0 Hz, 3H).

0.1 g of compound 4d and 0.15 g of compound 3a were added to a flask, and 10 mL of n-butanol and 10 mL of toluene were added. The flask was purged with nitrogen, and the reaction was carried out at 130 °C for 12 hours. The solvent was removed, and the residue was subjected to column chromatography to obtain 90 mg of Compound Ie, with a yield of 49%.

¹H NMR (500 MHz, Chloroform-*d*) δ 7.79 (ddd, *J*=10.6, 7.5, 2.0 Hz, 2H), 7.68-7.62 (m, 3H), 7.60 (dt, *J*=2.4, 0.9 Hz, 1H), 7.49 (q, *J*=7.5 Hz, 2H), 6.97 (dd, *J*=7.5, 1.5 Hz, 1H), 6.80 (dd, *J*=7.6, 1.6 Hz, 1H), 6.65-6.61 (m, 1H), 4.09 (q, *J*=8.0 Hz, 2H), 3.48 (q, *J*=8.0 Hz, 4H), 2.91-2.76 (m, 2H), 2.72 (t, *J*=7.1 Hz, 2H), 2.01 (p, *J*=7.1 Hz, 2H), 1.32 (t, *J*=8.0 Hz, 3H), 1.17 (t, *J*=8.0 Hz, 6H).

### Example 6: Synthesis of Compound If

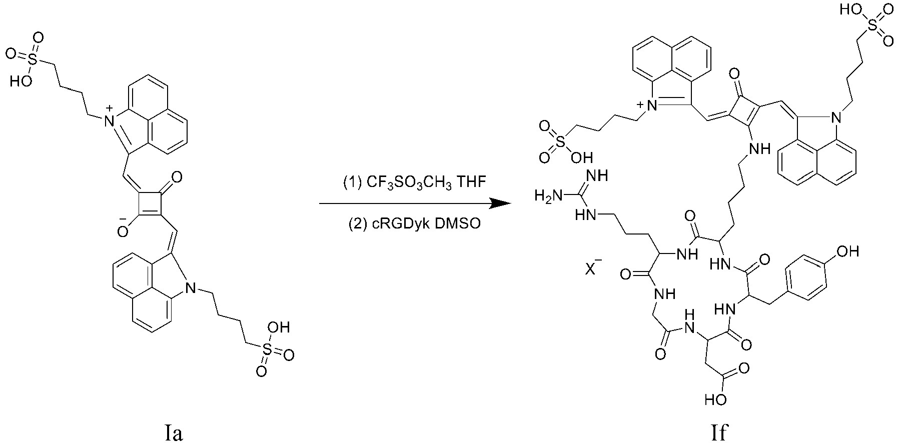

10 mg of compound Ia was dissolved in THF, and 100 µL of methyl trifluoromethanesulfonate was added. The mixture was stirred and reacted at room temperature for 5 hours. The reaction progress was monitored by HPLC. After the reaction was completed, the solvent was removed by rotary evaporation to give a residue, which was used directly in the next step. The residue was dissolved in anhydrous DMSO, followed by the addition 15 mg of cRGDyk peptide, and the reaction was carried out at room temperature for 12 hours. After the reaction was completed, the reaction solution was dialyzed using a dialysis bag, and further purified by C18 reverse-phase column chromatography. The final product was characterized by MALDI-TOF-MS.

MALDI-TOF-MS:
If: expected M.W: about 1286.46. Found: about 1286.5.

### Example 7: Synthesis of Compound Ig

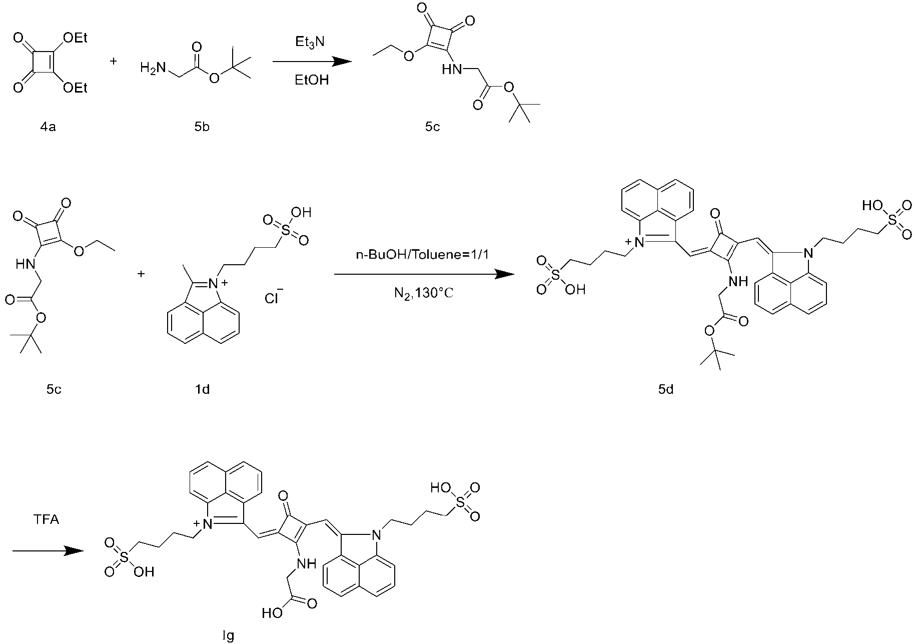

100 mg of compound 4a was dissolved in ethanol, and 77 mg of compound 5b and 76 mg of triethylamine were then added. The mixture was stirred and reacted at room temperature for 12 hours. The solvent was removed by rotary evaporation, and the residue was adsorbed onto silica gel and subjected to column chromatography to obtain compound 5c.

¹H NMR (600 MHz, Chloroform-*d*) δ 4.74 (q, *J*=7.1 Hz, 2H), 1.48 (s, 9H), 1.44 (t, *J*=7.1 Hz, 3H).

Compounds 5c and 1d were charged into a flask, and subsequently 10 mL of toluene and 10 mL of n-butanol were added. The flask was purged with nitrogen, and the reaction was carried out at 130 °C for 7 to 8 hours. The reaction was then discontinued, and the product was cooled to room temperature, and filtered to obtain compound 5d. Subsequently, compound 5d was placed in a flask, and TFA was added for deprotection. After the reaction was carried out for 5 to 6 hours, the solvent was removed, and the residue was subjected to reverse-phase column chromatography to obtain Compound Ig.

¹H NMR (400 MHz, DMSO-*d*₆) δ 10.00 (t, J=6.2 Hz, 1H), 8.83 (d, *J*=7.5 Hz, 1H), 8.32 (d, *J*=8.1 Hz, 1H), 8.25 (d, *J*=8.1 Hz, 1H), 8.18 (dd, *J*=7.6, 4.6 Hz, 1H), 8.00 (q, *J*=7.8 Hz, 2H), 7.89 (d, *J*=8.0 Hz, 1H), 7.84-7.61 (m, 5H), 6.79 (s, 1H), 6.43 (s, 1H), 5.75 (s, 1H), 4.71 (d, *J*=6.1 Hz, 2H), 4.39 (dt, *J*=26.7, 7.7 Hz, 4H), 2.62 (dt, *J*=19.7*,* 7.2 Hz, 4H), 2.07-1.97 (m, 2H), 1.96-1.71 (m, 6H).

¹³C NMR (126 MHz, DMSO) δ 174.3, 171.1, 169.7, 158.7, 157.6, 152.9, 151.5, 141.1, 140.8, 132.6, 132.3, 131.3, 130.2, 129.8, 129.6, 129.4, 129.3, 124.6, 124.4, 124.2, 122.9, 110.1, 93.8, 93.1, 51.1, 50.6, 46.0, 44.2, 44.0, 40.5, 40.3, 40.2, 40.0, 39.8, 39.7, 39.5, 27.9, 27.6, 22.9, 22.9.

### Example 8: Synthesis of Compound Ih

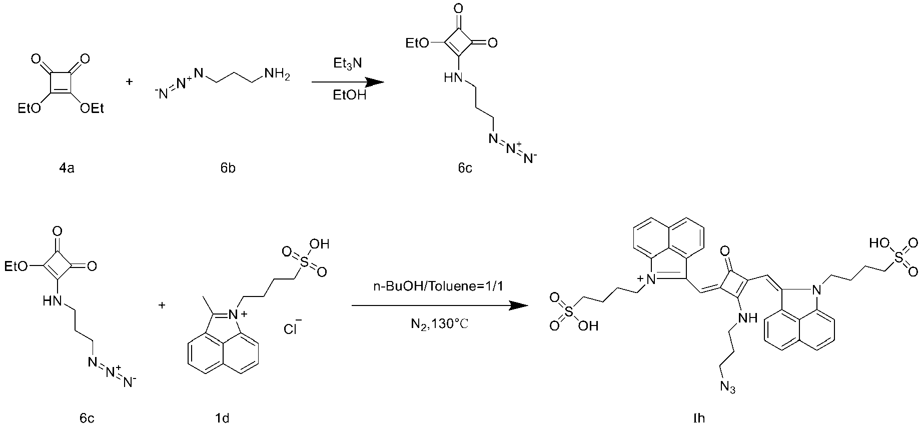

100 mg of compound 4a was dissolved in ethanol, and 60 mg of compound 6b and 76 mg of triethylamine were then added. The mixture was stirred and reacted at room temperature for 12 hours. The solvent was removed by rotary evaporation, and the residue was adsorbed onto silica gel and subjected to column chromatography to obtain compound 6c.

¹H NMR (600 MHz, Chloroform-*d*) δ 4.75 (q, *J*=7.1 Hz, 2H), 4.68 (s, 1H), 3.53 (q, *J*=6.6 Hz, 2H), 3.39 (t, *J*=6.4 Hz, 2H), 1.89 (q, *J*=6.6 Hz, 2H), 1.44 (t, *J*=7.1 Hz, 3H).

Compounds 6c and 1d were charged into a flask, and subsequently 10 mL of toluene and 10 mL of n-butanol were added. The flask was purged with nitrogen, and the reaction was carried out at 130 °C for 7 to 8 hours. The reaction was then discontinued, and the product was cooled to room temperature, filtered, and subjected to column chromatography to obtain Compound Ih.

¹H NMR (500 MHz, Chloroform-*d*) δ 7.82 (t, *J*=7.6 Hz, 1H), 7.74-7.65 (m, 2H), 7.60 (dt, *J*=7.5, 1.6 Hz, 1H), 7.58-7.53 (m, 3H), 7.50 (dd, *J*=7.5, 1.5 Hz, 1H), 7.50-7.45 (m, 2H), 7.47-7.37 (m, 1H), 7.16 (dd, *J*=7.3, 1.6 Hz, 1H), 6.74 (s, 1H), 6.62 (s, 1H), 6.44 (s, 2H), 6.10 (t, *J*=4.3 Hz, 1H), 4.63 (t, *J*=7.0 Hz, 2H), 4.16-4.08 (m, 2H), 3.24 (td, *J*=7.1, 4.3 Hz, 2H), 3.15 (t, *J*=7.1 Hz, 2H), 2.84 (t, *J*=7.1 Hz, 2H), 2.81-2.75 (m, 2H), 2.09 (pd, *J*=6.9, 0.7 Hz, 2H), 2.03-1.86 (m, 4H), 1.89-1.77 (m, 4H).

### Example 9: Ultraviolet Absorption Spectrum of Compound Ia

Compound Ia was dissolved separately in deionized water and methanol. 1.5 mL of each solution was taken and transferred into a quartz cuvette with a 1 cm path length. The absorption spectra of the different probes were measured on a UV2600 UV-Vis spectrophotometer, with the baseline corrected using the corresponding preparation solvent as a blank. The recording wavelength range was 300 nm to 1400 nm. The absorption peaks of compound Ia in water and methanol were at 738 nm and 858 nm, respectively. The experimental results indicated that compound Ia exhibits strong near-infrared absorption characteristics. In addition, the absorption spectra of compound Ia varied in different solvents, which may be attributed to the aggregation states of compound Ia in respective solvents.

### Example 10: Fluorescence Emission Spectrum of Compound Ia

Compound Ia was separately dissolved in deionized water and methanol. 200 µL of each solution was taken and transferred into a quartz cuvette with a 2 cm path length. The samples were excited by an 808 nm laser, and the emission wavelengths were recorded using an IHR320 fluorescence spectrometer over a wavelength measurement range from 825 nm to 1400 nm. The emission peak of compound Ia was at 925 nm, with its spectrum falling within the near-infrared II region. The experimental results demonstrated that compound Ia can emit near-infrared II light and is suitable for use as a near-infrared II fluorescent contrast agent.

### Example 11: In Vivo Biodistribution of Compound Ia in Normal Mice

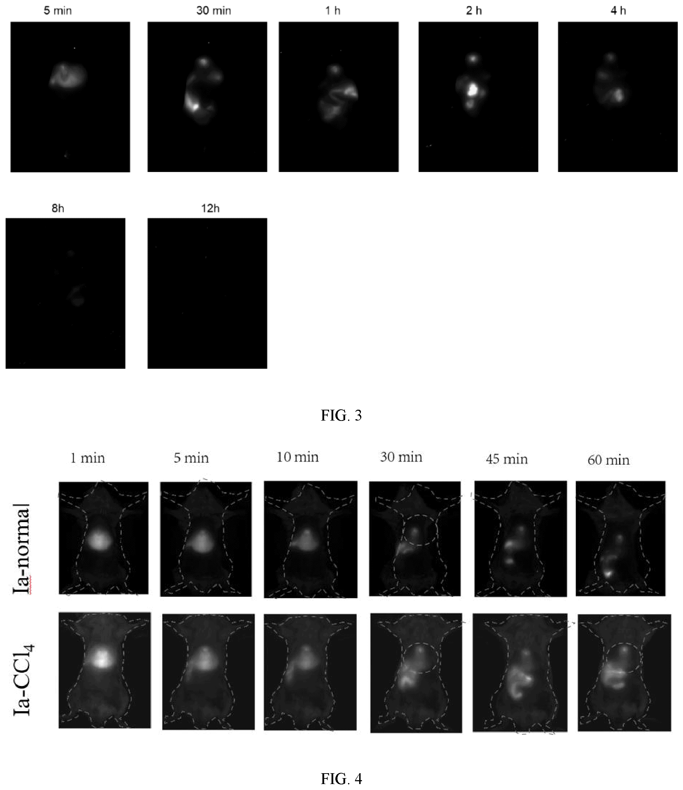

The mice used in the experiment were purchased upon approval from the Shanghai Laboratory Animal Research Center. Animal experiments were conducted in accordance with the guidelines of the Institutional Animal Care and Use Committee (IACUC) of the Shanghai Institute of Materia Medica, Chinese Academy of Sciences. Normal 6-week-old female Balb/c mice were used. Compound Ia was dissolved in PBS and injected into the mice via the tail vein. *In vivo* imaging was performed using a near-infrared II camera under excitation with an 808 nm laser and equipped with a 1000 nm long-pass filter. The results are shown in FIG. 3. FIG. 3 shows the biodistribution of compound Ia in the mice. The fluorescence intensity was mainly concentrated in the liver and intestines, indicating that the probe underwent hepatoenteral metabolism. Compound Ia exhibited rapid *in vivo* metabolism, no obvious toxicity, and good biocompatibility.

### Example 12: Experiment of Compound Ia in Carbon Tetrachloride-Induced Liver Injury Model Mice

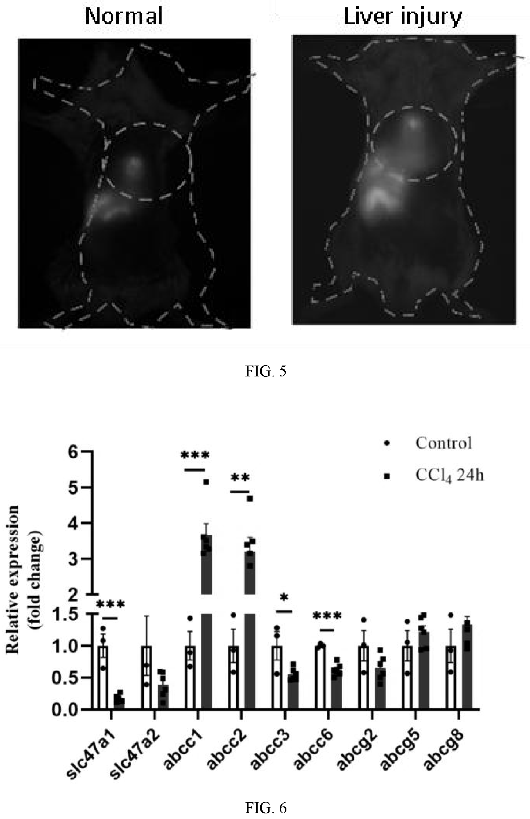

Male C57BL/6J mice aged 6 to 8 weeks with body weights of 18 to 22 g from the same batch were selected. Mice in the liver injury group were intraperitoneally injected with a 10% carbon tetrachloride (CCl₄) solution in olive oil at a dose of 2 mg/kg. Mice in the normal group were intraperitoneally injected with an equal volume of 0.9% normal saline. At 24 hours after CCl₄ treatment, blood samples were collected via retro-orbital bleeding. The activity of serum alanine aminotransferase (ALT) was measured as a standard indicator of liver function. The ALT concentration in the liver injury group increased sharply, indicating the successful establishment of the liver injury model. At 18 hours after carbon tetrachloride injection, Compound Ia dissolved in phosphate-buffered saline (PBS) was injected via the tail vein into each mouse of both the liver injury group and the control group. *In vivo* imaging was performed using a near-infrared II camera under excitation with an 808 nm laser and equipped with a 1100 nm long-pass filter. The results are shown in FIG. 4 and FIG. 5. Compared with the normal mice, the metabolism of Compound Ia in the carbon tetrachloride-induced liver injury model mice was significantly prolonged, indicating that compound Ia can be used for more rapid monitoring of early-stage liver injury.

### Example 13: Mechanistic Study of Carbon Tetrachloride-Induced Liver Injury Model Mice

The carbon tetrachloride-induced liver injury mouse model was established according to the procedure in Example 12, followed by anesthesia of the mice. Primary mouse hepatocytes were isolated, and qPCR assays were performed to test the gene expression levels of related efflux transporters in the liver tissues. The results are shown in FIG. 6. It can be observed that the expression levels of the *slc47a1, slc47a2, abcc3, abcc6,* and *abcg2* genes were all downregulated, with the most significant decrease observed in *slc47a1.* The experimental results indicate that liver injury impairs the function of hepatic efflux transporters, which may lead to reduced clearance capacities for metabolites and drugs, thereby delaying the metabolic process of compound Ia in the liver. In addition, by isolating primary mouse hepatocytes and constructing a cell injury model at the cellular level, qPCR results confirmed that *slc47a1* may be a potential target of Compound Ia.

### Example 14: Experiment of Compound Ia in slc47a1-Related MATE1 Transporter Inhibitor Model Mice

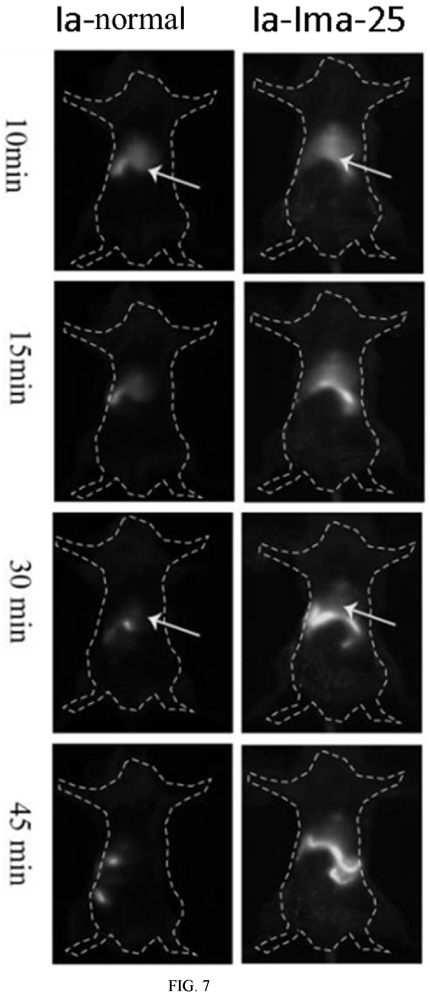

Male C57BL/6J mice aged 6 to 8 weeks old with body weights of 18 to 22 g from the same batch were selected. For the efflux-type MATE1 transporter inhibitor (imatinib) group, imatinib was first weighed according to a dose of 25 mg/kg or 50 mg/kg, dissolved in a small amount of DMSO for co-solubilization, and then diluted with a mixed solution of polyethylene glycol 300 and 1× PBS in a volume ratio of 1:1 to a final volume of 200 µL. At 30 min post-administration, 200 µL of a pre-prepared solution of compound Ia was injected. Fluorescence images of the abdomen of each mouse were captured at 1, 3, 5, 8, 10, 15, 25, 30, 45, 60, 90, and 120 min after injection of compound Ia. The imaging conditions were as follows: excitation wavelength of 808 nm, emission wavelength collected through an 1100 nm long-pass filter, and an exposure time of 200 ms. The results are shown in FIG. 7. Compared with the normal group, it was clearly observed that the metabolism of compound Ia in the liver of mice in the imatinib group was significantly inhibited and delayed at any time point of 10 min, 15 min, 30 min, or 45 min. The experimental results indicate that imatinib alters the metabolic process of compound Ia by inhibiting the *slc47a1*-encoded MATE1 transporter.

### Example 15: Photostability Experiment of Compound Ia

Indocyanine green (ICG), an FDA-approved near-infrared dye widely used clinically, was used as a control to evaluate the photostability of compound Ia (SQ 905). ICG was dissolved in water, and compound Ia (SQ 905) was dissolved in methanol. Over a period of 25 minutes, the methanol solution of compound Ia and the aqueous solution of ICG were continuously irradiated with an 808 nm laser at 108 mW/cm². During this period, the fluorescence intensity was recorded using a near-infrared II camera (MARS, Artemis Intelligent Imaging, Shanghai, China). Images were recorded every minute for the first 15 minutes, and every 5 minutes thereafter to monitor the fluorescence intensity. The fluorescence intensity of the region of interest (ROI) in the captured images was quantified using ImageJ software and compared with the initial fluorescence intensity to obtain the ratio of fluorescence intensity at each time point relative to the initial intensity. The photostability curve of compound Ia was plotted using GraphPad Prism 8.0.2 software.

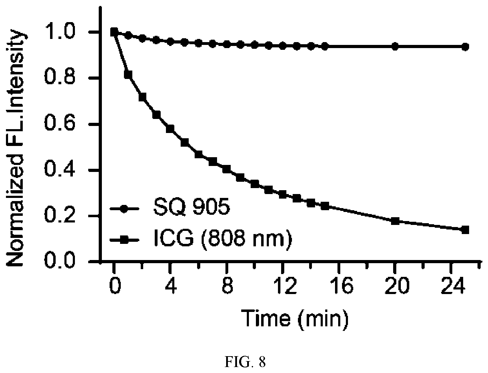

Photostability is an important indicator for probe evaluation. Insufficient photostability can lead to photobleaching and quenching during experiments, thereby compromising the quantitative fluorescence data and resulting in unreliable experimental results. Photostability substantially affects the subsequent clinical translation potential and application scenarios of probes. Accordingly, the photostability of compound Ia was evaluated in this example. The methanol solution of compound Ia and the aqueous solution of ICG were used for photostability testing. The results are shown in FIG. 8. After continuous irradiation with an 808 nm laser for 25 minutes, the fluorescence intensity of the methanol solution of compound Ia showed no significant attenuation. In contrast, the fluorescence intensity of the control ICG aqueous solution showed an 83% decrease after continuous irradiation with an 808 nm laser for 20 minutes. The data indicate that compound Ia exhibits significantly superior photostability compared with ICG, confirming its potential as a near-infrared II probe for subsequent biological applications.

Compounds Ib, Ic, Id, Ie, If, Ig, and Ih were respectively tested according to the methods described in Examples 9 to 15. The experimental results show that these compounds similarly exhibit strong near-infrared absorption characteristics and can emit near-infrared II light, making them suitable for use as near-infrared II fluorescent contrast agents. Furthermore, these compounds exhibited rapid *in vivo* metabolism, no obvious toxicity, good biocompatibility, and prominent photostability, demonstrating their suitability for more rapid monitoring of early-stage liver injury.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments are only several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the patent protection of the present application shall be defined by the appended claims.

## Claims

1. A squaric acid compound or a pharmaceutically acceptable salt or deuterated compound thereof, wherein the squaric acid compound is represented by a general structural formula I, II, III, IV, V, or VI: 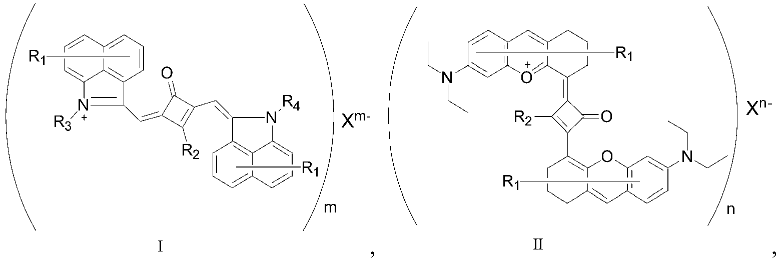 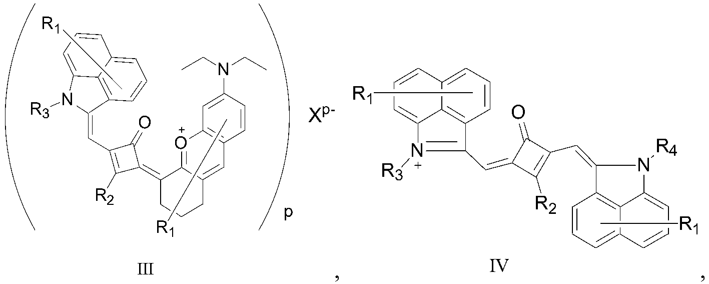 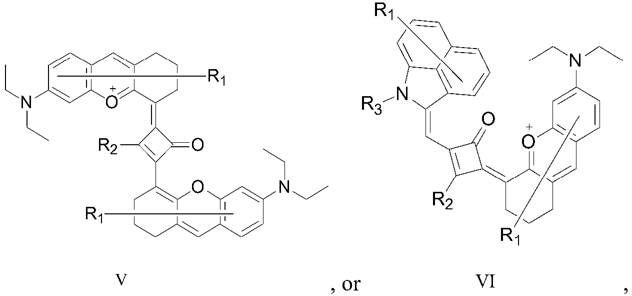
wherein X^{m-}, xⁿ⁻, and X^{p-} are each independently an anion,
m, n, and p are each independently any integer selected from 1 to 10;
each R₁ is independently at least one selected from the group consisting of H, C₁-C₁₂ alkyl, C₁-C₁₂ alkoxy, C₁-C₈ alkylsilyl, hydroxy C₁-C₈ alkyl, amino C₁-C₈ alkyl, formyl C₁-C₈ alkyl, mercapto C₁-C₈ alkyl, halo C₁-C₈ alkyl, acyloxy C₁-C₈ alkyl, amino, halogen, nitro, carboxy C₁-C₆ alkyl, substituted or unsubstituted C₆-C₁₀ aryl or 5 to 10 membered heteroaryl, -(CH₂)n₁-COOCH₂CH₂Si(CH₃)₃, -(CH₂)n₂-(CH₂CH₂O)n₃-R, and -(CH₂)n₂-(OCH₂CH₂)n₃-R;
R₂ is at least one selected from the group consisting of hydroxy, O⁻, C₁-C₁₂ alkoxy, C₁-C₈ alkylsilyl, hydroxy C₁-C₈ alkyl, carboxy, amino, -NR₅R₆, mercapto, -SR₇, and malononitrile;
R₃, R₄, R₅, R₆, and R₇ are each independently at least one selected from the group consisting of H, C₁-C₈ alkyl, hydroxy, amino, carboxy, C₁-C₁₂ alkyl, C₁-C₁₂ alkoxy, C₁-C₈ alkylsilyl, hydroxy C₁-C₈ alkyl, amino C₁-C₈ alkyl, aldehyde C₁-C₈ alkyl, mercapto C₁-C₈ alkyl, halo C₁-C₈ alkyl, acyloxy C₁-C₈ alkyl, amino, halogen, sulfo, carboxy C₁-C₆ alkyl, substituted or unsubstituted C₆-C₁₀ aryl or 5 to 10 membered heteroaryl, -(CH₂)n₁-COOCH₂CH₂Si(CH₃)₃, -(CH₂)n₂-(CH₂CH₂O)n₃-R, and -(CH₂)n₂-(OCH₂CH₂)n₃-R;
n1 is any integer from 0 to 10, n2 is any integer from 0 to 10, and n3 is any integer from 1 to 500;
R is at least one selected from the group consisting of H, C₁-C₈ alkyl, hydroxy, amino, carboxy, sulfo, halogen, mercapto, 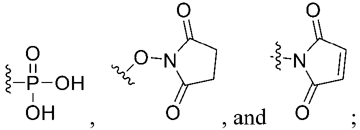 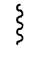
represents a linking site.

2. The squaric acid compound or a pharmaceutically acceptable salt or deuterated compound thereof according to claim 1, wherein the squaric acid compound is represented by a general structural formula I-1, II-1, III-1, IV-1, V-1, or VI-1:

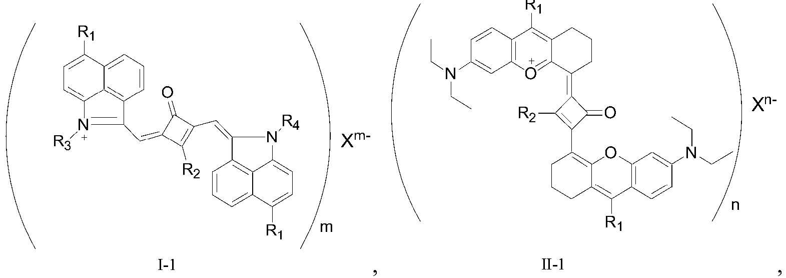

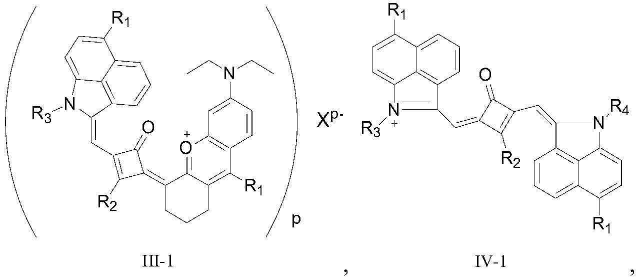

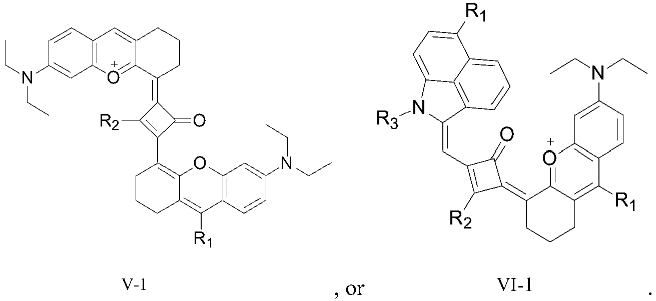

3. The squaric acid compound or a pharmaceutically acceptable salt or deuterated compound thereof according to claim 1 or 2, wherein a substituent of the substituted C₆-C₁₀ aryl or 5 to 10 membered heteroaryl is at least one selected from the group consisting of C₁-C₈ alkyl, C₁-C₈ alkoxy, C₁-C₈ alkylsilyl, hydroxy C₁-C₈ alkyl, amino C₁-C₈ alkyl, mercapto C₁-C₈ alkyl, halo C₁-C₈ alkyl, and carboxy C₁-C₆ alkyl.

4. The squaric acid compound or a pharmaceutically acceptable salt or deuterated compound thereof according to any one of claims 1 to 3, wherein m, n, and p are each independently any integer selected from 1 to 4.

5. The squaric acid compound or a pharmaceutically acceptable salt or deuterated compound thereof according to any one of claims 1 to 4, wherein m, n and p are each 1, and X⁻ is each independently selected from I⁻, Br⁻, BF₄⁻, or ClO₄⁻.

6. The squaric acid compound or a pharmaceutically acceptable salt or deuterated compound thereof according to any one of claims 1 to 5, wherein the squaric acid compound has any one of following structures:

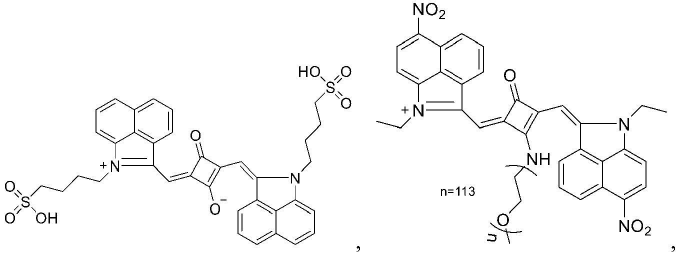

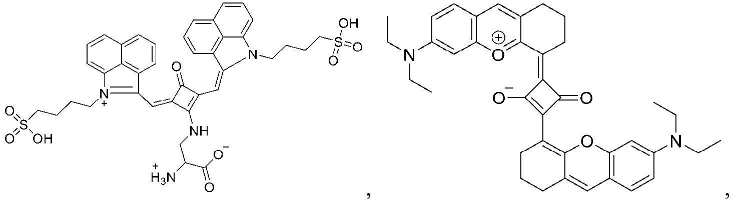

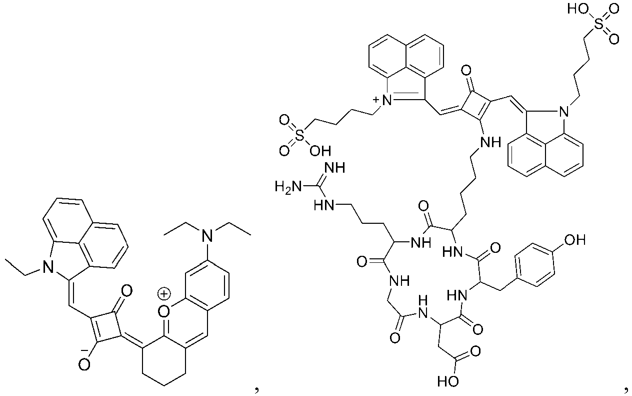

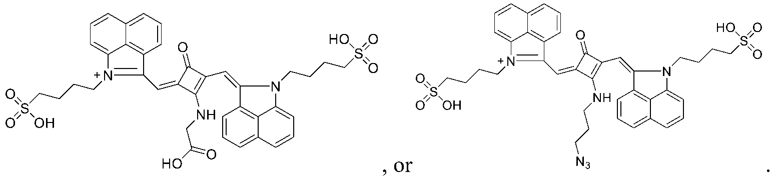

7. A method for preparing the squaric acid compound or the pharmaceutically acceptable salt or deuterated compound thereof according to any one of claims 1 to 6, comprising following steps:
(1) subjecting compound 1 and compound a-1 to a nucleophilic substitution reaction to obtain compound 2-1, the compound a-1 being halogenated R₃, and subjecting the compound 1 and compound a-2 to a nucleophilic substitution reaction to obtain compound 2-2, the compound a-2 being halogenated R₄;
subjecting the compound 2-1 and compound b to a Grignard reaction to obtain compound 3-1, and
subjecting the compound 2-2 and the compound b to a Grignard reaction to obtain compound 3-2, the compound b being a Grignard reagent;
subjecting the compound 3-1, the compound 3-2, and compound c to a condensation reaction to obtain compound 4, the compound c being squaric acid;
reacting compound 4 with compound d-1 to obtain the compound represented by formula I, the compound d-1 containing R₂; 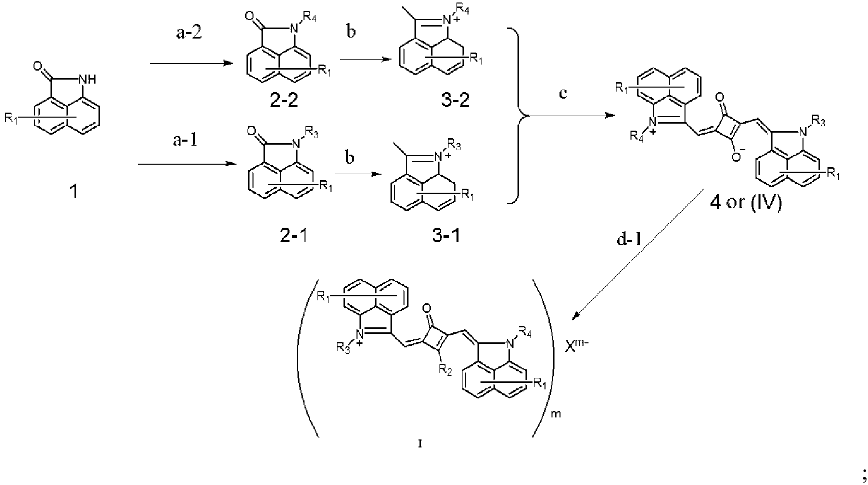 or
(2) subjecting compound 5 and compound c to a condensation reaction to obtain compound 6, the compound c being squaric acid;
reacting the compound 6 with compound d-2 to obtain the compound represented by formula II, the compound d-2 containing R₂; 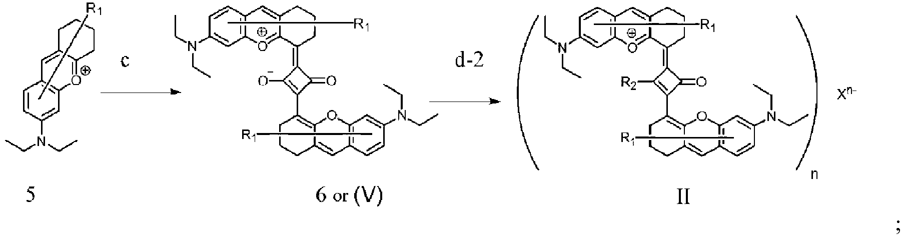 or
(3) subjecting compound 3-1 and compound e to a substitution reaction to obtain compound 7, the compound e being diethyl squarate;
subjecting the compound 7 and compound f to a hydrolysis reaction to obtain compound 8, the compound f being an alkali;
subjecting the compound 8 and compound 5 to a condensation reaction to obtain compound 9;
reacting the compound 9 with compound d-3 to obtain the compound represented by formula III, the compound d-3 containing R₂; 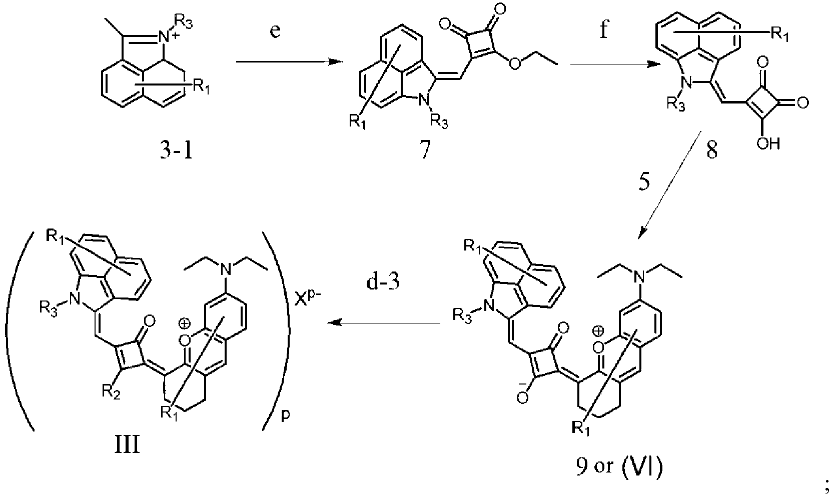
wherein R₁, R₂, R₃, and R₄ are defined as in any one of claims 1 to 6.

8. The method for preparing the squaric acid compound or the pharmaceutically acceptable salt or deuterated compound thereof according to claim 7, satisfying at least one of following (1) to (9):
(1) a molar ratio of the compound 1 to the compound a-1 is in a range from 1:1 to 1:1.5, and a molar ratio of the compound 1 to the compound a-2 is in a range from 1:1 to 1:1.5;
(2) a molar ratio of the compound 2-1 to the compound b is in a range from 1:1 to 1:1.5, and a molar ratio of the compound 2-2 to the compound b is in a range from 1:1 to 1:1.5;
(3) a molar ratio of the compound 3-1 to the compound c is in a range from 1:1 to 3:1, and a molar ratio of the compound 3-2 to the compound c is in a range from 1:1 to 3:1;
(4) a molar ratio of the compound 4 to the compound d-1 is in a range from 1:1 to 1:1.5;
(5) a molar ratio of the compound 5 to the compound c is in a range from 2:1 to 3:1;
(6) a molar ratio of the compound 6 to the compound d-2 is in a range from 1:1 to 1:1.5;
(7) a molar ratio of the compound 3-1 to the compound e is in a range from 1:1 to 1.5:1;
(8) a molar ratio of the compound 8 to the compound 5 is in a range from 1:1 to 1:1.5;
(9) a molar ratio of the compound 9 to the compound d-3 is in a range from 1:1 to 1:1.5.

9. A fluorescent probe, comprising the squaric acid compound or the pharmaceutically acceptable salt or deuterated compound thereof according to any one of claims 1 to 6.

10. An NIR-II contrast agent, comprising the squaric acid compound or the pharmaceutically acceptable salt or deuterated compound thereof according to any one of claims 1 to 6.

11. A method for monitoring early liver injury, comprising imaging a subject in need thereof using the squaric acid compound or the pharmaceutically acceptable salt or deuterated compound thereof according to any one of claims 1 to 6, the fluorescent probe according to claim 9, or the NIR-II contrast agent according to claim 10.
